# EUROPEAN PATENT APPLICATION

(11) **EP 4 726 391 A1**
(43) Date of publication of application: **15.04.2026**
(21) Application number: 24205915.2
(22) Date of filing: 10.10.2024
(51) Int. Cl.: G01N 33/564, C07K 14/47

(54) **NOVEL CITRULLINATED PEPTIDES THAT BIND TO ANTI-CITRULLINATED PROTEIN ANTIBODIES (ACPAS) AND THEIR USE IN THE DIAGNOSIS AND PREDICTION OF RHEUMATOID ARTHRITIS**

(71) Applicant: Vacara AB, 171 65 Solna (SE)
(72) Inventor: He, Yibo, 171 65 Solna (SE); Sareila, Outi Talvikki, 171 65 Solna (SE); Martinsson Holmdahl, David Rikard, 171 65 Solna (SE)
(74) Representative: karo IP

(57) **Abstract**

The disclosure relates to novel citrullinated polypeptides that bind to anti-citrullinated protein antibodies (ACPAs) and their use in the diagnosis and prediction of rheumatoid arthritis.

## Description

### Background

Rheumatoid arthritis (RA) is a chronic autoimmune disorder that primarily affects the joints, leading to inflammation, pain, and progressive joint destruction. It is well-established that RA involves an immune response against citrullinated proteins, where anti-citrullinated protein antibodies (ACPAs) play a significant role. The detection of these antibodies, particularly through assays using cyclic citrullinated peptides (CCP), has become a crucial tool in diagnosing RA, as ACPAs can be present in individuals long before the onset of clinical symptoms. Citrullination, a post-translational modification where arginine residues are converted into citrulline, generates epitopes that are recognized by ACPAs, marking the modified proteins for immune attack.

Despite the advances in ACPA detection and the widespread use of CCP-based assays, the current state of diagnostic and prognostic methods for RA still faces several limitations. Current biomarkers, while useful in confirming a diagnosis of RA, are not fully effective in the prognosis of RA patients or predicting the future risk of developing RA, especially in individuals who are asymptomatic. Furthermore, conventional ACPA assays do not account for the functional heterogeneity of ACPAs-some ACPAs may have pathogenic effects, while others have been observed to exert protective roles in certain models. Thus, current diagnostic tools fail to distinguish between protective and pathogenic ACPA responses, limiting their utility in providing a comprehensive understanding of disease risk or progression.

### Summary

Certain autoantibodies, such as anti-citrullinated protein antibodies (ACPAs) and rheumatoid factors (RF), are present in the circulation of individuals years before the onset of RA. Their repertoire and titre increase as the disease onset approaches. Certain citrullinated peptides, such as citrullinated α-enolase peptide 1 (CEP1), jointprotein 5 (JP5), and CCP, have been identified as significant epitopes in RA. These peptides are recognized by ACPAs and have been studied for their role in disease development. However, research has shown that not all ACPA responses to these peptides are pathogenic. For example, it has been observed that a subset of ACPAs (herein termed 'protective ACPAs'), such as clone E4, can form protective immune complexes with citrullinated peptides, potentially interacting with immune receptors like Fcgr2b on macrophages (He *et al.,* 2023; He *et al.,* 2024). This protective effect highlights the need for more refined biomarkers that can distinguish between different ACPA subsets; *i.e.,* protective vs non-protective ACPAs and their respective roles in disease modulation.

It is therefore an objective of the present disclosure to provide novel polypeptide biomarkers that improve the prediction of future risk, diagnosis, and prognosis of RA. These biomarkers will allow for the identification of a subset of said protective ACPAs and their epitope recognition patterns, thereby offering a more precise evaluation of disease risk, severity, progression, and overall activity.

The present disclosure generally provides for citrullinated peptides and groups thereof, derived from alpha-enolase (ENO1) and Peptidyl arginine deiminase 4 (PAD4), and their use in methods for the future risk prediction, prognosis and diagnosis of rheumatoid arthritis (RA).

In one aspect, the present disclosure provides for a polypeptide comprising or consisting of a sequence selected from the group consisting of:
SEQ ID NO: 3: SKAKFAG-Cit-NFRNPLA
SEQ ID NO: 4: VSPPVTV-Cit-GKEYPLG
SEQ ID NO: 5: VRVFQAT-Cit-GKLSSK, and
SEQ ID NO: 6: DFFTYHI-Cit-HGEVHCG

In one aspect, the present disclosure relates to a plurality of polypeptides comprising at least two polypeptides of the disclosure.

The polypeptides of the present disclosure provide technical advantages in their ability to serve as biomarkers for rheumatoid arthritis (RA), in the contexts of future risk, diagnosis, and prognosis of RA. These polypeptides have been specifically identified to bind to anti-citrullinated protein antibodies (ACPAs), which are a hallmark of RA. The recognition of these polypeptides by ACPAs indicates that they are likely to be present before the onset of clinical RA symptoms, thus providing a valuable tool for early detection of individuals at risk of developing RA.

In one aspect, the polypeptides of the invention may be used in the diagnosis of a subject, prognosis of an individual or identification of an individual at risk of developing an autoimmune disease. The use of these specific polypeptides allows for more precise identification of antibody responses in subjects, facilitating an earlier diagnosis and providing insights into the disease's progression and activity.

The polypeptides of the invention may be used in methods for diagnosing RA, distinguishing between different types or stages of RA, prognosing the disease, or identifying individuals at risk of developing RA.

In one aspect, the present disclosure provides for a method of diagnosing rheumatoid arthritis in a subject, the method comprising detecting whether at least one anti-citrullinated protein antibody (ACPA) present in a sample provided from the subject or individual, binds to at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120.

In one aspect, the present disclosure provides for a method of prognosing rheumatoid arthritis in an individual or identifying an individual at risk of developing rheumatoid arthritis, the method comprising detecting whether at least one anti-citrullinated protein antibody (ACPA) present in a sample provided from the subject or individual, binds to at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120.

The invention provides an improved means of stratifying patients based on the detection of specific antibody responses, which may correspond to either protective or pathogenic ACPA responses. This method allows for the identification of antibodies that not only indicate disease presence but also provide prognostic information regarding disease severity and activity, enabling a more personalised approach to treatment. Additionally, the polypeptides enable a diagnostic method that can be employed before the onset or within the first year of clinical symptoms, allowing for earlier intervention. By providing the polypeptides, the invention presents the potential to distinguish between different RA disease phenotypes based on the binding profiles of ACPAs to the polypeptides. This differentiation may be used to predict future risk and guide therapeutic decisions. The methods disclosed may be implemented in various clinical and research settings where early detection and prognosis of RA is required, offering both diagnostic and prognostic capabilities that enhance patient care.

In one aspect, the present disclosure provides for a kit of parts comprising:
a. at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the at least one polypeptide is immobilized on a surface; and,
b. at least one detection reagent selected from the group comprising enzyme-conjugated antibodies, fluorescence-conjugated antibodies, or luminescent probes for detecting antibody binding to the at least one polypeptide

### Description of Figures

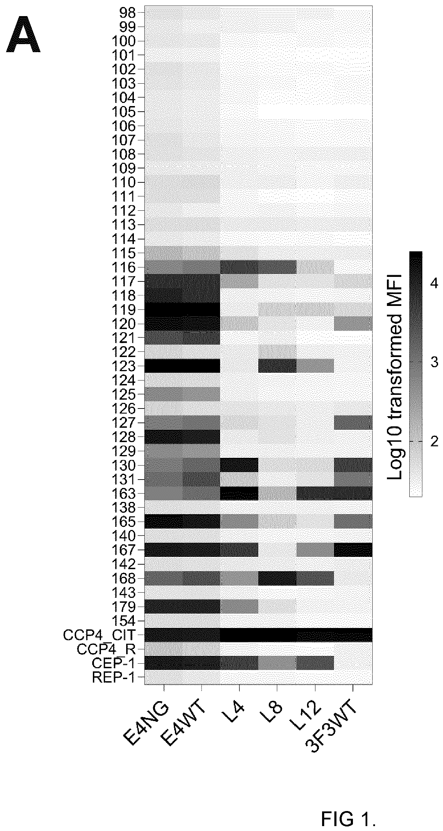

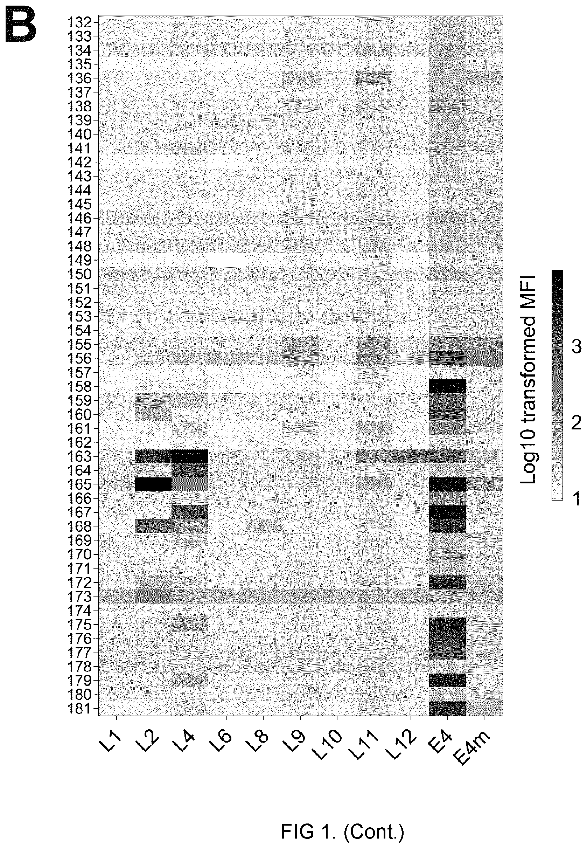

**Figure 1. A)** and **B)** **Reactivity of monoclonal antibodies (ACPAs) to citrullinated cyclic peptides derived from human alpha enolase (ENO1) and peptidylarginine deiminase 4 (PAD4).** X axis denotes tested polypeptides. Antibodies denoted on the X axis. The heatmaps represent the log10-transformed multiplex bead-based flow immunoassay (MFI) results at the antibody concentration of 1.0 µg/ml. E4, L4, L8, L12 and 3F3WT are monoclonal ACPA derived from RA patients. E4 denotes the E4 antibody; NG denotes non-glycosylated; WT denotes wild-type.

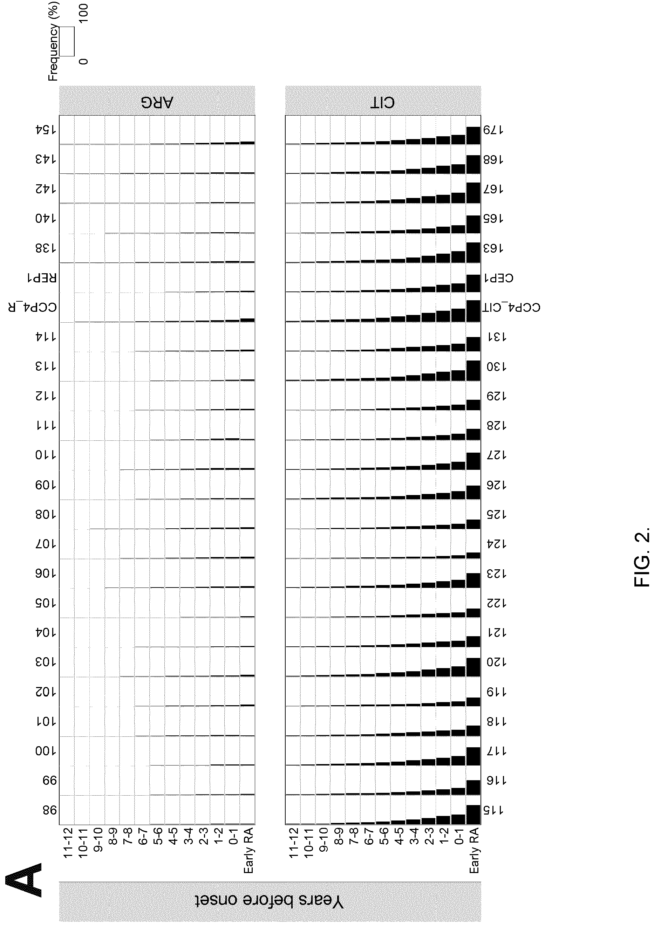

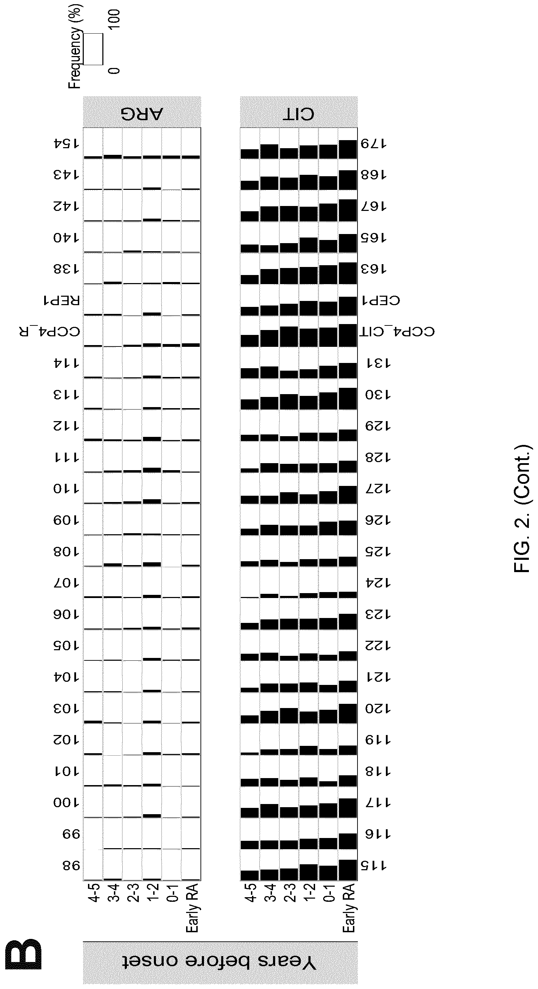

**Figure 2****. IgG responses to citrullinated (CIT) or native/unmodified (R) of ENO1 and PAD4-derived peptides in pre-symptomatic individuals and early RA patients. A)** Cumulative frequency of antibody responses to ENO1 and PAD4-derived peptides in plasma samples from the pre-symptomatic individuals (n= 520) at timepoints before the onset of clinical symptoms (from 12 years to less than 1 year before onset of clinical symptoms), and in early RA (within 0-1 years from the onset of clinical symptoms; n= 244). **B)** Individual frequency of antibody responses to ENO1 and PAD4-derived peptides in plasma samples from the pre-symptomatic individuals from 6 to less than 1 year before symptom onset (n= 335), and in early RA (within 0-1 years after the onset of clinical symptoms; n= 244). The cut-off for positivity was determined as 95% specificity in population controls. Y axis denotes tested polypeptides Y-axis is frequency of positive IgG responses (%). Response to CCP4,
CEP1 and their respective non-citrulline control peptides (CCP4_R and REP1) are shown for comparison.

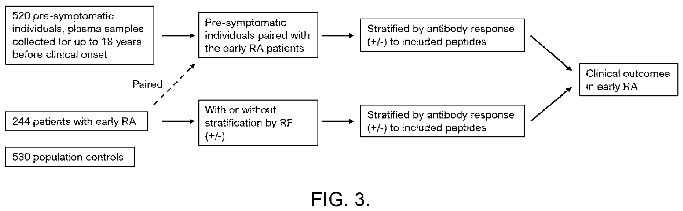

**Figure 3****.** Flowchart showing the analysis steps for association between antibody response and early RA clinical outcomes in pre-symptomatic individuals or patients with early RA.

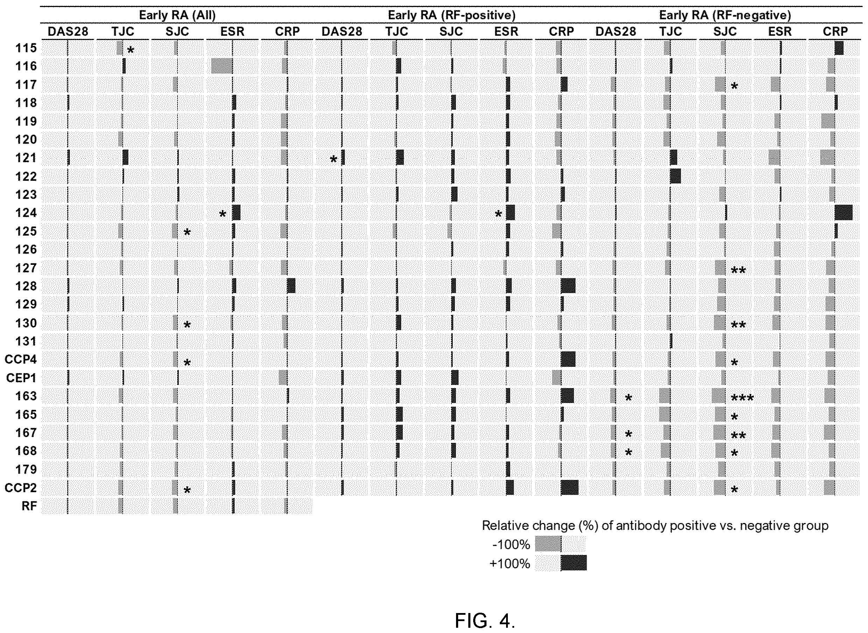

**Figure 4****. Differences in clinical presentation of early RA patients based on serological biomarkers.** Relative change in mean clinical presentation (DAS28, TJC, SJC, ESR and CRP) of early RA patients (N=244) positive for the serological biomarkers compared to those negative for the biomarkers when tested at the early RA stage (within 0-1 years after onset of clinical symptoms). Serological biomarkers denote IgG ACPA responses against tested citrullinated peptides. Y axis denotes the SEQ ID NOs of tested polypeptides and the anti-CCP2 (CCP2) and RF (denoted on Y axis). Results are shown as relative change (%) to the mean of the biomarker negative group. Decrease indicates a less severe disease in biomarker positive group. **p*<0.05, ***p*<0.01 and ****p*<0.001. DAS28 denotes Disease-activity score-28; TJC denotes tender joint count, SJC denotes swollen joint count; ESR denotes erythrocyte sedimentation rate; CRP denotes C-reactive protein. 'All' denotes all early RA patients, not stratified via RF status.

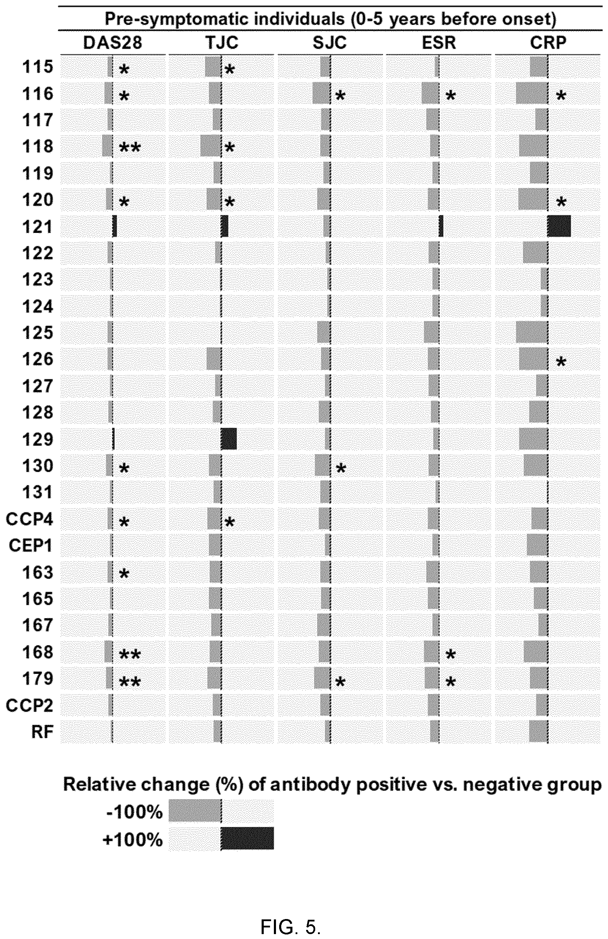

**Figure 5****. Differences in clinical presentation of early RA patients based on serological biomarkers determined at the pre-symptomatic stage.** Relative change in mean clinical presentation (DAS28, TJC, SJC, ESR and CRP) of early RA patients (within 0-1 year after the onset of clinical symptoms; N=117 for DAS28 and ESR; N=116 for SJC and TJC; and N=82 for CRP) positive for the serological biomarkers compared to those negative for the biomarker when tested at their pre-symptomatic stage (0-5 years before the onset of clinical RA symptoms). Serological biomarkers include IgG ACPA responses against tested citrullinated peptides (Y axis denotes the SEQ ID Nos of each tested citrullinated polypeptide) as well as the anti-CCP2 (CCP2) and RF. Results are shown as relative change (%) to the mean of the biomarker negative group. A decrease in relative change in mean clinical presentation indicates a less severe disease in biomarker positive group compared with the biomarker negative group. **p*<0.05, ***p*<0.01. DAS28 denotes Disease-activity score-28; TJC denotes tender joint count, SJC denotes swollen joint count; ESR denotes erythrocyte sedimentation rate; CRP denotes C-reactive protein.

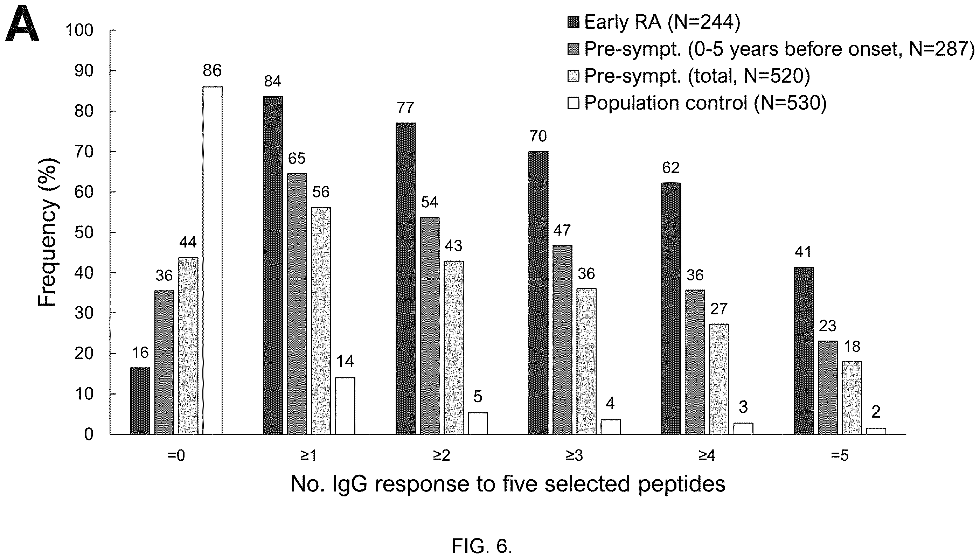

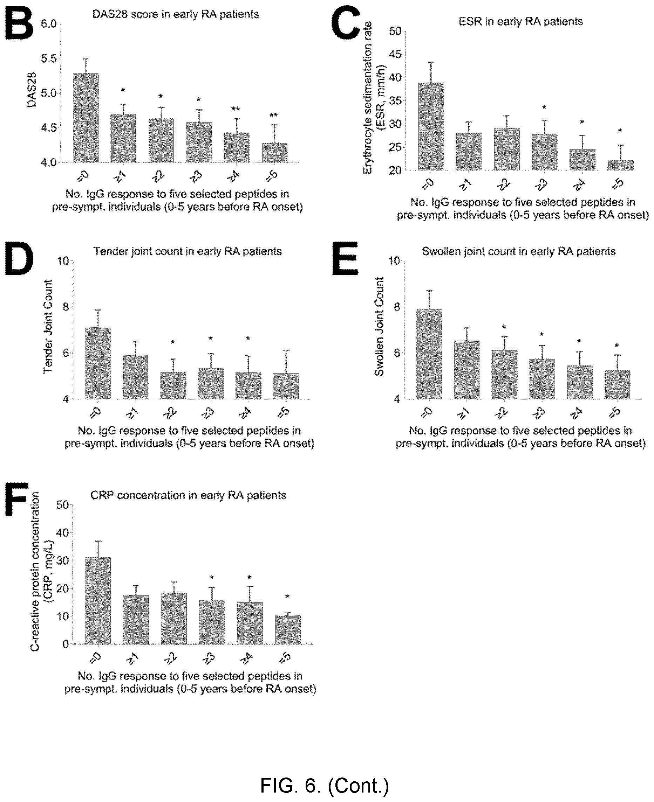

**Figure 6****. Diagnostic and predictive value of the new ACPA test. A)** Frequencies of ACPA IgG responses to five peptides (SEQ ID NO 120, SEQ ID NO 130, SEQ ID NO 163, SEQ ID NO 168 and SEQ ID NO 179) , the new ACPA test, in early RA patients, pre-symptomatic individuals and population control. **B-F)** Clinical outcomes of early RA patients (B; DAS28 score, C; ESR, D; TJC, E; SJC, F; CRP concentration) associated with the number of ACPA responses in the same individuals at the pre-symptomatic stage (0-5 years before onset; N=117 for (B) DAS28 score,(D) TJC, (E) SJC and (C) ESR; and N=82 for (F) CRP). For each criterium, the comparisons were separately made between the individuals with ≥1, ≥2, ≥3, ≥4, or =5 positive ACPA responses to any of the five peptides vs. the individuals with no response to any of the peptides (=0). Data were analysed with Mann-Whitney U test (two-tailed) and presented as mean ± SEM. DAS; disease activity score. TJC; tender joint count. SJC; swollen joint count. ESR; erythrocyte sedimentation rate. CRP; C-reactive protein. **p*<0.05, ***p*<0.01.

### Detailed description

### Definitions

As used herein, the singular forms "a", "an" and "the" include plural referents unless the context clearly states otherwise. Thus, for example, reference to "an antibody" includes a plurality of such constructs.

The term "some embodiments" can include one, or more than one embodiment.

As used herein, the term 'absent', when used in reference to an amino acid and/or amino acid sequence, is taken to mean that the residues on either side of the 'absent' or 'absence' are connected by a peptide bond.

An "amino acid residue" or "amino acid" can be a natural or non-natural amino acid residue linked by peptide bonds or bonds different from peptide bonds. The amino acid residues can be in D-configuration or L-configuration. An amino acid residue comprises an amino terminal part (NH2) and a carboxy terminal part (COOH) separated by a central part comprising a carbon atom, or a chain of carbon atoms, at least one of which comprises at least one side chain or functional group. NH2 refers to the amino group present at the amino terminal end of an amino acid or peptide, and COOH refers to the carboxy group present at the carboxy terminal end of an amino acid or peptide. The generic term amino acid comprises both natural and non-natural amino acids. Natural amino acids of standard nomenclature as listed in J. Biol. Chem., (1969; 243:3552-59) and adopted in 37 C.F.R., section 1.822(b)(2) belong to the group of amino acids listed herewith: Y,G,F,M,A,S,I,L,T,V,P,K,H,Q,E,W,R,D,N and C. Non-natural amino acids are those not listed immediately above. Also, non-natural amino acid residues include, but are not limited to, modified amino acid residues, L-amino acid residues, and stereoisomers of D-amino acid residues.

As used herein, the term "an individual at risk" refers to a person, subject and/or individual who, based on specific factors, has an increased likelihood of developing a particular disease or disorder compared to the general population. These factors may include, but are not limited to, the presence of specific biomarkers, genetic predisposition, family history of related conditions, environmental exposures, lifestyle factors, or early clinical indicators that suggest susceptibility to the disease or disorder. The individual may not yet display overt symptoms of the disease but is considered to have a heightened probability of developing the condition in the future. This term applies to various diseases or disorders, including but not limited to autoimmune diseases such as rheumatoid arthritis.

As used herein, the term "Anti-Citrullinated Protein Antibodies" or "ACPAs" or "ACPA" refers to autoantibodies that specifically recognize proteins or peptides that contain the non-standard amino acid citrulline. ACPAs are commonly associated with autoimmune conditions, particularly rheumatoid arthritis, and can be used as biomarkers for the diagnosis and prognosis of such diseases. ACPAs are well-known in the art. Examples of ACPAs include, but are not limited to E4, as described in US 11,866,512.

As used herein, the term "Autoimmune Disease" refers to a pathological condition and/or disease and/or disorder and/or syndrome in which the body's immune system erroneously targets and attacks its own tissues, cells, or organs, recognizing them as foreign. This misdirected immune response leads to chronic inflammation, tissue destruction, and impaired function of the affected organs or systems. Autoimmune diseases can affect a wide range of tissues, including joints, skin, nerves, and internal organs, and may result in a variety of symptoms depending on the specific disease. Common examples include, but are not limited to, rheumatoid arthritis, systemic lupus erythematosus, multiple sclerosis, and type 1 diabetes.

As used herein, the term "Citrulline" (also referred to as "Cit") refers to a non-standard amino acid with the chemical formula C₆H₁₃N₃O₃, which results from the post-translational modification of the amino acid arginine through a process called citrullination or deimination. This enzymatic reaction, catalysed by peptidylarginine deiminase (PAD), converts the positively charged guanidinium group of arginine into a neutral urea group, thereby altering the charge and hydrophobicity of the polypeptide. Citrulline is of particular significance in autoimmune diseases, especially rheumatoid arthritis (RA), where it is recognized by anti-citrullinated protein antibodies (ACPAs). As used herein, the term "Cyclic polypeptide" refers to a polypeptide in which the N-terminal and C-terminal ends, or two internal amino acid residues, are covalently linked to form a circular, closed-loop structure. This covalent bond can be formed through peptide bonds, disulfide bridges between cysteine residues, or other types of chemical linkages such as lactamization or head-to-tail cyclization. Cyclic polypeptides often exhibit enhanced stability, resistance to proteolytic degradation, and altered biological activity compared to their linear counterparts. The term encompasses both naturally occurring cyclic polypeptides and synthetic or engineered cyclic forms, which may include non-natural amino acids, modified residues, or chemical modifications to enhance their functional properties.

As used herein, the term "Detection System" refers to any method or apparatus used to identify or quantify the presence or binding of a molecule, such as an antibody, to a target, such as a polypeptide. Detection systems include, but are not limited to, enzyme-linked immunosorbent assays (ELISA), fluorescence-based assays, chemiluminescence assays, or radioisotope-based detection, which provide a measurable signal upon binding.

As used herein, the term "diagnosis" refers to determining with respect to an individual: whether the individual has a disease or not and/or how severe the disease of the individual is and/or prognosis of the disease of the individual and/or expected response to treatment of the individual and/or or risk of developing the disease, classification of the disease, monitoring the progression of the disease or the results of intervention."

As used herein "fragment", when used in reference to an antibody, is a polypeptide having an amino acid sequence that is the same as part of but not all of the amino acid sequence of the reference antibody.

As used herein, the phrase "has a length of between X and Y amino acid residues" is taken to mean a polypeptide consisting of a number of amino acid residues X to Y both thresholds included, i.e. the polypeptide may consist of X amino acid residues, Y amino acid residues, or a number of amino acid residues falling between X and Y, but it cannot consist of more than Y amino acid residues. By way of example, a polypeptide has a length of between of 3 to 6 amino acid residues consists of exactly 3, 4, 5, or 6 amino acid residues.

As used herein, the term "immobilized" refers to a molecule, such as a polypeptide, that is covalently or non-covalently attached to a surface, preventing it from freely moving in solution. The surface may be, but is not limited to, solid or semi-solid. The immobilization may occur via chemical linkages or affinity-based interactions, allowing the molecule to be retained on the surface for further use in detection assays or other applications.

The term "individual" refers to vertebrates, particular members of the mammalian species, preferably primates including humans. As used herein, 'subject' and 'individual' may be used interchangeably. Treatment of animals, such as mice, rats, dogs, cats, cows, horses, sheep and pigs, is, however, also within the scope of the present invention.

As used herein, the term "linking moiety" refers to a chemical group or spacer that covalently connects two molecules, such as a polypeptide and a detection tag or another molecule. The linking moiety can be composed of various chemical structures, such as aminohexanoic acid (Ahx), polyethylene glycol (PEG), or short peptide chains, and serves to preserve the functionality of the linked molecules while allowing flexibility or spatial separation.

As used herein, the term "Net neutral charge" refers to the overall electrical charge of a molecule, such as a polypeptide, where the total number of positively charged groups equals the total number of negatively charged groups, resulting in no overall electrical charge at physiological pH or under specific conditions.

As used herein, the term "Net positive charge" refers to the overall electrical charge of a molecule, such as a polypeptide, where the total number of positively charged groups (e.g., lysine or arginine residues) exceeds the total number of negatively charged groups (e.g., aspartic acid or glutamic acid residues), resulting in an overall positive charge at physiological pH or under specific conditions.

As used herein, the terms "protein", "polypeptide" and "peptide" are used interchangeably and refer to a polymer mainly consisting of multiple amino acids linked by peptide bonds, without reference to a specific mode of action, size, 3-dimensional structure or origin. A fragment or portion of a protein may thus still be referred to as a "protein". An "isolated protein" is used to refer to a protein which is no longer in its natural environment, for example in vitro. The terms encompass sequences of amino acids of any length, including short sequences often referred to as peptides, as well as longer chains typically considered proteins. The terms include naturally occurring, recombinant, and synthetic polypeptides, which may contain standard L-amino acids, non-natural amino acids, D-amino acids, or chemically modified residues. A polypeptide may also include modifications such as phosphorylation, acetylation, glycosylation, cyclization, or the incorporation of disulfide bridges and can exhibit a variety of structural forms, including linear, branched, or cyclic configurations. For the purpose of this application, a "polypeptide" can be defined by a specific amino acid sequence (e.g., SEQ ID NO), and may include any derivatives, fragments, or analogues thereof.

As used herein, the term "prognosis" refers to the prediction of the likely course, outcome, or severity of a disease in a subject. Prognosis provides information on the expected progression of the condition and may help guide treatment decisions.

As used herein, the term "sample" refers to any biological material obtained from an individual, which may contain anti-citrullinated protein antibodies (ACPAs) or fragments thereof, and is used for the detection, diagnosis, or analysis of an autoimmune disease such as rheumatoid arthritis. The sample may include, but is not limited to, serum, plasma, saliva, synovial fluid, or any other biological fluid, tissue, or cell preparation. The sample can be processed and analysed using standard laboratory techniques to detect binding interactions with the polypeptides described herein.

As used herein, the term "full-length proteins" refers to proteins that are expressed or synthesized in their entirety, including the complete sequence of amino acids from the N-terminus to the C-terminus as found in the native or naturally occurring form of the protein. In the context of the present disclosure, full-length proteins such as human alpha enolase (ENO1; SEQ ID NO: 96) and peptidyl arginine deiminase 4 (PAD4; SEQ ID NO: 97) provide the basis for deriving polypeptide sequences. These full-length proteins include all domains and structural features present in the naturally occurring protein, allowing for the identification of sites, such as arginine residues, that may undergo post-translational modifications like citrullination.

As used herein, the term "derivative thereof" refers to any modification, variant, or version of a substance that is structurally related to the original substance while retaining a significant portion of its chemical identity or functional characteristics. In the context of molecules, including biomolecules such as peptides, proteins, or small molecules, a derivative may involve substitutions, additions, deletions, or other chemical modifications. These changes may result in functional enhancements, improved stability, altered solubility, or modified binding properties while maintaining a degree of similarity to the original substance that allows it to perform a related function.

### Linear sequences

In one aspect, the invention provides for a polypeptide comprising or consisting of the sequence
X₁ - X₂ - X₃ - X₄ - X₅ - X₆ - X₇ - Cit - X_{8 -} X₉ - X₁₀ - X₁₁ - X₁₂ - X₁₃ - X₁₄ (SEQ ID NO: 1)
X₁ is Serine (S), Valine (V), Aspartic Acid (D) or Glutamic Acid (E),
X₂ is Lysine (K), Serine (S), Arginine (R) or Phenylalanine (F),
X₃ is Alanine (A), Proline (P), Valine (V), Phenylalanine (F) or Glycine (G),
X₄ is Lysine (K), Proline (P), Phenylalanine (F), Threonine (T) or Valine (V),
X₅ is Phenylalanine (F), Valine (V), Glutamine (Q), Tyrosine (Y) or Proline (P),
X₆ is Alanine (A), Threonine (T), Histidine (H) or Leucine (L),
X₇ is Glycine (G), Valine (V), Threonine (T), Isoleucine (I) or Tyrosine (Y),
X₈ is Asparagine (N), Glycine (G) or Histidine (H),
X₉ is Phenylalanine (F), Lysine (K), Glycine (G) or Isoleucine (I),
X₁₀ is Arginine (R), Glutamic Acid (E), Alanine (A) or absent,
X₁₁ is Asparagine (N), Tyrosine (Y), Leucine (L), Valine (V) or Aspartic Acid (D),
X₁₂ is Proline (P), Serine (S), Histidine (H) or Leucine (L),
X₁₃ is Leucine (L), Serine (S), or Alanine (A),
X₁₄ is Alanine (A), Glycine (G) or Lysine (K).

In one embodiment, the polypeptide comprises or consists of the sequence
X₁ - X₂ - X₃ - X₄ - X₅ - X₆ - X₇ - Cit - X_{8 -} X₉ - X₁₀ - X₁₁ - X₁₂ - X₁₃ - X₁₄ (SEQ ID NO: 1)
X₁ is Serine (S), Valine (V) or Aspartic Acid (D),
X₂ is Lysine (K), Serine (S), Arginine (R) or Phenylalanine (F),
X₃ is Alanine (A), Proline (P), Valine (V) or Phenylalanine (F),
X₄ is Lysine (K), Proline (P), Phenylalanine (F) or Threonine (T),
X₅ is Phenylalanine (F), Valine (V), Glutamine (Q) or Tyrosine (Y),
X₆ is Alanine (A), Threonine (T) or Histidine (H),
X₇ is Glycine (G), Valine (V), Threonine (T) or Isoleucine (I),
X₈ is Asparagine (N), Glycine (G) or Histidine (H),
X₉ is Phenylalanine (F), Lysine (K) or Glycine (G),
X₁₀ is Arginine (R), Glutamic Acid (E) or absent,
X₁₁ is Asparagine (N), Tyrosine (Y), Leucine (L) or Valine (V),
X₁₂ is Proline (P), Serine (S) or Histidine (H),
X₁₃ is Leucine (L), or Serine (S),
X₁₄ is Alanine (A), Glycine (G) or Cysteine (C).

In one embodiment, the polypeptide comprises or consists of the sequence
X₁ - X₂ - X₃ - X₄ - X₅ - X₆ - X₇ - Cit - X_{8 -} X₉ - X₁₀ - X₁₁ - X₁₂ - X₁₃ - X₁₄ (SEQ ID NO: 1)
X₁ is Serine (S) or Valine (V),
X₂ is Lysine (K), Serine (S) or Arginine (R),
X₃ is Alanine (A), Proline (P) or Valine (V),
X₄ is Lysine (K), Proline (P) or Phenylalanine (F),
X₅ is Phenylalanine (F), Valine (V) or Glutamine (Q),
X₆ is Alanine (A) or Threonine (T),
X₇ is Glycine (G), Valine (V) or Threonine (T),
X₈ is Asparagine (N) or Glycine (G),
X₉ is Phenylalanine (F) or Lysine (K),
X₁₀ is Arginine (R) or Glutamic Acid (E) or absent,
X₁₁ is Asparagine (N), Tyrosine (Y) or Leucine (L),
X₁₂ is Proline (P) or Serine (S),
is Leucine (L) or Serine (S),
X₁₄ is Alanine (A), Glycine (G) or Lysine (K).

In one embodiment, the polypeptide comprises or consists of the sequence
X₁ - X₂ - X₃ - X₄ - X₅ - X₆ - X₇ - Cit - X_{8 -} X₉ - X₁₀ - X₁₁ - P - L - X₁₄ (SEQ ID NO: 2)
X₁ is Serine (S) or Valine (V),
X₂ is Lysine (K) or Serine (S),
X₃ is Alanine (A) or Proline (P),
X₄ is Lysine (K) or Proline (P),
X₅ is Phenylalanine (F) or Valine (V),
X₆ is Alanine (A) or Threonine (T),
X₇ is Glycine (G) or Valine (V),
X₈ is Asparagine (N) or Glycine (G),
X₉ is Phenylalanine (F) or Lysine (K),
X₁₀ is Arginine (R) or Glutamic Acid (E),
X₁₁ is Asparagine (N) or Tyrosine (Y),
X₁₂ is Alanine (A) or Glycine (G).

In one embodiment, the polypeptide comprises or consists of the sequence:
A - Cit - B
wherein A comprises an amino acid sequence consisting of 8 contiguous amino acid residues selected from the group consisting of SKAKFAG (SEQ ID NO; 21); VSPPVTV (SEQ ID NO; 22); VRVFQAT (SEQ ID NO; 23); DFFTYHI (SEQ ID NO; 24); EKGVPLY (SEQ ID NO; 25); and
wherein B comprises an amino acid sequence consisting of between 7 and 9 contiguous amino acid residues selected from the group consisting of NFRNPLA (SEQ ID NO; 26); GKEYPLG (SEQ ID NO; 27); GKLSSK (SEQ ID NO; 28); HGEVHCG (SEQ ID NO; 29); HIADLAG (SEQ ID NO; 30).

In one embodiment, the polypeptide is selected from the group consisting of:
SEQ ID NO: 3: SKAKFAG-Cit-NFRNPLA
SEQ ID NO: 4: VSPPVTV-Cit-GKEYPLG
SEQ ID NO: 5: VRVFQAT-Cit-GKLSSK
SEQ ID NO: 6: DFFTYHI-Cit-HGEVHCG, and
SEQ ID NO: 7: EKGVPLY-Cit-HIADLAG

In one embodiment, the polypeptide is selected from the group consisting of:
SEQ ID NO: 3: SKAKFAG-Cit-NFRNPLA
SEQ ID NO: 4: VSPPVTV-Cit-GKEYPLG
SEQ ID NO: 5: VRVFQAT-Cit-GKLSSK, and
SEQ ID NO: 6: DFFTYHI-Cit-HGEVHCG.

In one embodiment, the polypeptide is SEQ ID NO: 7: EKGVPLY-Cit-HIADLAG. In one embodiment, the polypeptide is SEQ ID NO: 3: SKAKFAG-Cit-NFRNPLA. In one embodiment, the polypeptide is SEQ ID NO: 4: VSPPVTV-Cit-GKEYPLG. In one embodiment, the polypeptide is SEQ ID NO: 5: VRVFQAT-Cit-GKLSSK. In one embodiment, the polypeptide is SEQ ID NO: 6: DFFTYHI-Cit-HGEVHCG.

### Cyclic sequences

In one aspect, the invention provides for a polypeptide comprising or consisting of the sequence:
C - X₁ - X₂ - X₃ - X₄ - X₅ - X₆ - X₇ - Cit - X_{8 -} X₉ - X₁₀ - X₁₁ - X₁₂ - X₁₃ - X₁₄ - X₁₅ - X₁₆ (SEQ ID NO: 8)
X₁ is Serine (S), Valine (V), Aspartic Acid (D) or Glutamic Acid (E),
X₂ is Lysine (K), Serine (S), Arginine (R) or Phenylalanine (F),
X₃ is Alanine (A), Proline (P), Valine (V), Phenylalanine (F) or Glycine (G),
X₄ is Lysine (K), Proline (P), Phenylalanine (F), Threonine (T) or Valine (V),
X₅ is Phenylalanine (F), Valine (V), Glutamine (Q), Tyrosine (Y) or Proline (P),
X₆ is Alanine (A), Threonine (T), Histidine (H) or Leucine (L),
X₇ is Glycine (G), Valine (V), Threonine (T), Isoleucine (I) or Tyrosine (Y),
X₈ is Asparagine (N), Glycine (G) or Histidine (H),
X₉ is Phenylalanine (F), Lysine (K), Glycine (G) or Isoleucine (I),
X₁₀ is Arginine (R), Glutamic Acid (E), Alanine (A) or absent,
X₁₁ is Asparagine (N), Tyrosine (Y), Leucine (L), Valine (V) or Aspartic Acid (D),
X₁₂ is Proline (P), Serine (S), Histidine (H) or Leucine (L),
is Leucine (L), Serine (S), Cysteine (C) or Alanine (A),
X₁₄ is Alanine (A), Glycine (G) or Lysine (K),
X₁₅ is absent or Cysteine (C),
X₁₆ is absent or Alanine (A).

In one embodiment, the polypeptide comprises or consists of the sequence
C - X₁ - X₂ - X₃ - X₄ - X₅ - X₆ - X₇ - Cit - X_{8 -} X₉ - X₁₀ - X₁₁ - X₁₂ - X₁₃ - X₁₄ - X₁₅ - X₁₆ (SEQ ID NO: 8)
X₁ is Serine (S), Valine (V) or Aspartic Acid (D),
X₂ is Lysine (K), Serine (S), Arginine (R) or Phenylalanine (F),
X₃ is Alanine (A), Proline (P), Valine (V) or Phenylalanine (F),
X₄ is Lysine (K), Proline (P), Phenylalanine (F) or Threonine (T),
X₅ is Phenylalanine (F), Valine (V), Glutamine (Q) or Tyrosine (Y),
X₆ is Alanine (A), Threonine (T) or Histidine (H),
X₇ is Glycine (G), Valine (V), Threonine (T) or Isoleucine (I),
X₈ is Asparagine (N), Glycine (G) or Histidine (H),
X₉ is Phenylalanine (F), Lysine (K) or Glycine (G),
X₁₀ is Arginine (R), Glutamic Acid (E) or absent,
X₁₁ is Asparagine (N), Tyrosine (Y), Leucine (L) or Valine (V),
X₁₂ is Proline (P), Serine (S) or Histidine (H),
is Leucine (L), Serine (S) or Cysteine (C),
X₁₄ is Alanine (A), Glycine (G) or Cysteine (C),
X₁₅ is absent or Cysteine (C),
X₁₆ is absent or Alanine (A).

In one embodiment, the polypeptide comprises or consists of the sequence
C - X₁ - X₂ - X₃ - X₄ - X₅ - X₆ - X₇ - Cit - X_{8 -} X₉ - X₁₀ - X₁₁ - X₁₂ - X₁₃ - X₁₄ - C- A (SEQ ID NO: 9)
X₁ is Serine (S) or Valine (V),
X₂ is Lysine (K), Serine (S) or Arginine (R),
X₃ is Alanine (A), Proline (P) or Valine (V),
X₄ is Lysine (K), Proline (P) or Phenylalanine (F),
X₅ is Phenylalanine (F), Valine (V) or Glutamine (Q),
X₆ is Alanine (A) or Threonine (T),
X₇ is Glycine (G), Valine (V) or Threonine (T),
X₈ is Asparagine (N) or Glycine (G),
X₉ is Phenylalanine (F) or Lysine (K),
X₁₀ is Arginine (R) or Glutamic Acid (E) or absent,
X₁₁ is Asparagine (N), Tyrosine (Y) or Leucine (L),
X₁₂ is Proline (P) or Serine (S),
is Leucine (L) or Serine (S),
X₁₄ is Alanine (A), Glycine (G) or Lysine (K).

In one embodiment, the polypeptide comprises or consists of the sequence
C - X₁ - X₂ - X₃ - X₄ - X₅ - X₆ - X₇ - Cit - X_{8 -} X₉ - X₁₀ - X₁₁ - P - L - X₁₄ - C - A (SEQ ID NO: 10)
X₁ is Serine (S) or Valine (V),
X₂ is Lysine (K) or Serine (S),
X₃ is Alanine (A) or Proline (P),
X₄ is Lysine (K) or Proline (P),
X₅ is Phenylalanine (F) or Valine (V),
X₆ is Alanine (A) or Threonine (T),
X₇ is Glycine (G) or Valine (V),
X₈ is Asparagine (N) or Glycine (G),
X₉ is Phenylalanine (F) or Lysine (K),
X₁₀ is Arginine (R) or Glutamic Acid (E),
X₁₁ is Asparagine (N) or Tyrosine (Y),
X₁₂ is Alanine (A) or Glycine (G).

In one embodiment, the polypeptide comprises or consists of the sequence:
A - Cit - B
wherein A comprises an amino acid sequence consisting of 8 contiguous amino acid residues selected from the group consisting of CSKAKFAG (SEQ ID NO; 31); CVSPPVTV (SEQ ID NO; 32); CVRVFQAT (SEQ ID NO; 33); CDFFTYHI (SEQ ID NO; 34); CEKGVPLY (SEQ ID NO; 35); and
wherein B comprises an amino acid sequence consisting of between 7 and 9 contiguous amino acid residues selected from the group consisting of NFRNPLACA (SEQ ID NO; 36); GKEYPLGCA (SEQ ID NO; 37); GKLSSKCA (SEQ ID NO; 38); HGEVHCG (SEQ ID NO; 39); HIADLAGCA (SEQ ID NO; 40).

In one embodiment, the polypeptide is selected from the group consisting of:
SEQ ID NO: 11: CSKAKFAG-Cit-NFRNPLACA
SEQ ID NO: 12: CVSPPVTV-Cit-GKEYPLGCA
SEQ ID NO: 13: CVRVFQAT-Cit-GKLSSKCA
SEQ ID NO: 14: CDFFTYHI-Cit-HGEVHCG, and
SEQ ID NO: 15: CEKGVPLY-Cit-HIADLAGCA

In one embodiment, the polypeptide is selected from the group consisting of:
SEQ ID NO: 11: CSKAKFAG-Cit-NFRNPLACA
SEQ ID NO: 12: CVSPPVTV-Cit-GKEYPLGCA
SEQ ID NO: 13: CVRVFQAT-Cit-GKLSSKCA, and
SEQ ID NO: 14: CDFFTYHI-Cit-HGEVHCG.

In one embodiment, the polypeptide is SEQ ID NO: 15: CEKGVPLY-Cit-HIADLAGCA. In one embodiment, the polypeptide is SEQ ID NO: 11: CSKAKFAG-Cit-NFRNPLACA. In one embodiment, the polypeptide is SEQ ID NO: 12: CVSPPVTV-Cit-GKEYPLGCA. In one embodiment, the polypeptide is SEQ ID NO: 13: CVRVFQAT-Cit-GKLSSKCA. In one embodiment, the polypeptide is SEQ ID NO: 14: CDFFTYHI-Cit-HGEVHCG.

In one embodiment, the polypeptide consists of between 12 to 20 amino acid residues, such as between 13 to 20 amino acid residues, such as between 14 to 20 amino acid residues, such as between 15 to 20 amino acid residues, such as between 13 to 19 amino acid residues, such as between 13 to 18 amino acid residues, such as between 13 to 18 amino acid residues, such as between 14 to 18 amino acid residues, such as between 14 to 17 amino acid residues, such as between 14 to 16 amino acid residues, such as between 14 to 15 amino acid residues.

In one embodiment, the polypeptide consists of between 14 to 18 amino acid residues. In one embodiment, the polypeptide consists of between 14 to 15 amino acid residues.

In one embodiment, the polypeptide the polypeptide has a net positive charge. In some embodiments, the polypeptide has a net positive charge and is selected from the group consisting of: SEQ ID NO: 3: SKAKFAG-Cit-NFRNPLA, and SEQ ID NO: 5: VRVFQAT-Cit-GKLSSK. In one embodiment, the polypeptide has a net positive charge. In some embodiments, the polypeptide has a net neutral charge and is selected from the group consisting of: SEQ ID NO: 4: VSPPVTV-Cit-GKEYPLG, SEQ ID NO: 6: DFFTYHI-Cit-HGEVHCG, and SEQ ID NO: 7: EKGVPLY-Cit-HIADLAG. In one embodiment, the polypeptide is hydrophobic.

### Binding to ACPAs

The inclusion of citrulline (Cit) at specific position of the polypeptides of the invention allows for recognition and binding by antibodies, such as anti-citrullinated protein antibodies (ACPAs), particularly in the context of autoimmune diseases such as rheumatoid arthritis. The polypeptide may serve as an epitope for detecting such antibodies, facilitating the diagnosis or prognosis of autoimmune conditions.

In one embodiment, the polypeptide binds to at least one anti-citrullinated protein antibody (ACPA) or fragment thereof. In some embodiments, the at least one anti-citrullinated protein antibody or fragment thereof comprises a variable heavy chain (VH) domain and a variable light chain (VL) domain, and wherein
a. the VH domain comprises an amino acid sequence that includes complementarity determining regions (CDRs):
   i. a HCDR1 sequence comprising an amino acid sequence as set forth in SEQ ID NO: 45; a HCDR2 sequence comprising an amino acid sequence as set forth in SEQ ID NO: 46; and a HCDR3 sequence comprising an amino acid sequence as set forth in SEQ ID NO: 43 or 47, or
   ii. a HCDR1 sequence comprising an amino acid sequence as set forth in SEQ ID NO: 61; a HCDR2 sequence comprising an amino acid sequence as set forth in SEQ ID NO: 62; and a HCDR3 sequence comprising an amino acid sequence as set forth in SEQ ID NO: 63, or
   iii. a HCDR1 sequence comprising an amino acid sequence as set forth in SEQ ID NO: 70; a HCDR2 sequence comprising an amino acid sequence as set forth in SEQ ID NO: 71; and a HCDR3 sequence comprising an amino acid sequence as set forth in SEQ ID NO: 72, or
   iv. a HCDR1 sequence comprising an amino acid sequence as set forth in SEQ ID NO: 90; a HCDR2 sequence comprising an amino acid sequence as set forth in SEQ ID NO: 91; and a HCDR3 sequence comprising an amino acid sequence as set forth in SEQ ID NO: 92, and
b. the VL domain comprises an amino acid sequence that includes complementarity determining regions (CDRs):
   i. a LCDR1 sequence comprising an amino acid sequence as set forth in SEQ ID NO: 48; a LCDR2 sequence comprising an amino acid sequence as set forth in SEQ ID NO: 49; and a LCDR3 sequence comprising an amino acid sequence as set forth in SEQ ID NO: 44 or 50, or
   ii. a LCDR1 sequence comprising an amino acid sequence as set forth in SEQ ID NO: 53; a LCDR2 sequence comprising an amino acid sequence as set forth in SEQ ID NO: 54; and a LCDR3 sequence comprising an amino acid sequence as set forth in SEQ ID NO: 55, or
   iii. a LCDR1 sequence comprising an amino acid sequence as set forth in SEQ ID NO: 79; a LCDR2 sequence comprising an amino acid sequence as set forth in SEQ ID NO: 80; and a LCDR3 sequence comprising an amino acid sequence as set forth in SEQ ID NO: 81, or
   iv. a LCDR1 sequence comprising an amino acid sequence as set forth in SEQ ID NO: 93; a LCDR2 sequence comprising an amino acid sequence as set forth in SEQ ID NO: 94; and a LCDR3 sequence comprising an amino acid sequence as set forth in SEQ ID NO: 95.

As used herein, the term "HCDR" (heavy chain complementarity determining region) refers to the regions within the variable domain of the heavy chain (VH) of an antibody that are responsible for binding to an antigen. These regions are highly variable and contribute to the antigen specificity of the antibody. The heavy chain complementarity determining regions (HCDRs) include HCDR1, HCDR2, and HCDR3, with each region being defined by specific sequences within the VH domain. In the present context, the HCDR1, HCDR2, and HCDR3 sequences are represented by SEQ ID NOs, which define the specific amino acid sequences involved in recognizing and binding to citrullinated epitopes, such as those in anti-citrullinated protein antibodies (ACPAs).

As used herein, the term "LCDR" (light chain complementarity determining region) refers to the regions within the variable domain of the light chain (VL) of an antibody that are also involved in antigen recognition and binding. Similar to the HCDRs, the light chain complementarity determining regions (LCDRs) include LCDR1, LCDR2, and LCDR3, which are defined by specific amino acid sequences within the VL domain. The LCDR sequences described in this context, as set forth by SEQ ID NOs, are part of the overall structure that interacts with the antigen, providing specificity to the binding of ACPAs. Together, the HCDRs and LCDRs form the antigen-binding site of the antibody, determining its specificity for the target antigen.

In some embodiments, the at least one anti-citrullinated protein antibody or fragment thereof comprises:
a. a variable heavy chain (VH) domain comprising an amino acid sequence selected from SEQ ID NO: 52, SEQ ID NO: 69, SEQ ID NO: 78 or SEQ ID NO: 88; and
b. a variable light chain (VL) domain comprising an amino acid sequence selected from SEQ ID NO: 51, SEQ ID NO: 60, SEQ ID NO: 87 or SEQ ID NO: 89.

In some embodiments, the at least one anti-citrullinated protein antibody or fragment thereof comprises a variable heavy chain (VH) domain and a variable light chain (VL) domain, and wherein
a. the VH domain comprises an amino acid sequence that includes complementarity determining regions (CDRs):
   i. a HCDR1 sequence comprising an amino acid sequence as set forth in SEQ ID NO: 61; a HCDR2 sequence comprising an amino acid sequence as set forth in SEQ ID NO: 62; and a HCDR3 sequence comprising an amino acid sequence as set forth in SEQ ID NO: 63; or
   ii. a HCDR1 sequence comprising an amino acid sequence as set forth in SEQ ID NO: 70; a HCDR2 sequence comprising an amino acid sequence as set forth in SEQ ID NO: 71; and a HCDR3 sequence comprising an amino acid sequence as set forth in SEQ ID NO: 72; and
b. the VL domain comprises an amino acid sequence that includes complementarity determining regions (CDRs):
   i. a LCDR1 sequence comprising an amino acid sequence as set forth in SEQ ID NO: 53; a LCDR2 sequence comprising an amino acid sequence as set forth in SEQ ID NO: 54; and a LCDR3 sequence comprising an amino acid sequence as set forth in SEQ ID NO: 55, or
   ii. a LCDR1 sequence comprising an amino acid sequence as set forth in SEQ ID NO: 79; a LCDR2 sequence comprising an amino acid sequence as set forth in SEQ ID NO: 80; and a LCDR3 sequence comprising an amino acid sequence as set forth in SEQ ID NO: 81.

In some embodiments, the at least one anti-citrullinated protein antibody or fragment thereof comprises:
a. a variable heavy chain (VH) domain comprising or consisting of an amino acid sequence selected from SEQ ID NO: 69 or SEQ ID NO: 78; and
b. a variable light chain (VL) domain comprising or consisting of an amino acid sequence selected from SEQ ID NO: 60 or SEQ ID NO: 87.

In some embodiments, the at least one anti-citrullinated protein antibody or fragment thereof is present in the sample of an individual with an autoimmune disease. In some embodiments, the autoimmune disease is rheumatoid arthritis. In some embodiments, the sample is selected from the group comprising serum, plasma, saliva and synovial fluid.

As used herein, the terms "antibody responses" and "antibody reactivity" and "ACPA responses" are used interchangeably to refer to the binding or association between antibodies, such as IgGs and/or anti-citrullinated protein antibodies (ACPAs), and specific antigens or epitopes, particularly citrullinated antigens, in a biological sample. The biological sample may include the serum of a patient or individual. In the context of rheumatoid arthritis (RA), these terms encompass the detection and binding of antibodies to specific citrullinated residues or polypeptides. This recognition may correlate with disease activity, progression, or clinical outcomes, and includes the concept of "fine specificities". The skilled person would be knowledgeable of techniques to determine and quantify "antibody responses" as defined herein, using methods such as those disclosed in the examples. Relevant analytical methods include multiplex bead-based flow immunoassays, such as the one described in Example 2, which allow for the simultaneous detection of multiple antibody specificities within a single sample. Enzyme-linked immunosorbent assays (ELISA) are also widely used to quantify antibody-antigen interactions by measuring the binding of antibodies to immobilized citrullinated peptides or proteins. Surface plasmon resonance (SPR) enables real-time monitoring of the binding kinetics and affinity between antibodies and their specific antigens. Other relevant methods include Western blotting (or immunoblotting), where antibodies can be detected based on their ability to bind to citrullinated antigens separated by gel electrophoresis. Electrochemiluminescence assays offer sensitive detection and quantification of antibody responses by combining electrochemical and luminescent signals. Finally, immunohistochemistry or immunofluorescence can be employed to visualize the binding of antibodies to citrullinated epitopes in tissue samples. These methods, individually or in combination, enable the skilled person to detect and quantify antibody responses and ACPA reactivity with high specificity and sensitivity.

As used herein, the term "fine specificities" refers to the precise and detailed recognition by antibodies, such as anti-citrullinated protein antibodies (ACPAs), of specific epitopes or structural features on a target antigen. In the context of rheumatoid arthritis (RA), "fine specificities" indicates the ability of ACPAs to bind to distinct citrullinated polypeptides or specific citrullinated residues within a polypeptide sequence, thereby differentiating between various citrullinated antigens. This term encompasses the nuanced interactions between ACPAs and particular citrullinated epitopes, which may correlate with specific disease outcomes or clinical features.

As used herein, the term 'serological biomarkers' or 'serological biomarker' are measurable substances found in the blood, often antibodies or proteins, that can indicate the presence of a disease, infection, or immune response. These biomarkers are used to assess and monitor disease states, help in diagnosis, predict outcomes, or guide treatment decisions. The terms may refer to the IgG ACPA (anti-citrullinated protein antibodies) responses against citrullinated peptides, as well as the anti-CCP2 (cyclic citrullinated peptide 2) antibodies and rheumatoid factor (RF). These biomarkers are used to compare clinical outcomes in early RA patients by determining if individuals who test positive for these biomarkers (at the pre-symptomatic or early RA stage) show different levels of disease severity, as measured by clinical indicators such as DAS28, TJC, SJC, ESR, and CRP. A decrease in relative change in clinical presentation indicates that individuals who are serological biomarker positive exhibit a less severe disease outcome compared to those who are serological biomarker negative. In essence, these biomarkers are used to assess whether the presence of certain antibodies correlates with milder disease symptoms in early RA.

As used herein, the terms `clinical anti-CCP2 test', also referred to as the 'CCP2-test' or 'anti-CCP2 test' or 'CCP-based assays', is a widely used standard clinical laboratory test for detecting anti-cyclic citrullinated peptide antibodies (ACPAs) in a patient's blood (Aletaha *et al.,* 2010; Smolen *et al.,* 2023). These antibodies that specifically bind to specifically second-generation cyclic citrullinated peptides (CCP2). which are citrullinated forms of proteins commonly produced in individuals with rheumatoid arthritis (RA). The presence of ACPAs, as detected by the anti-CCP2 test, is highly specific to RA and serves as a biomarker in diagnosing the disease, particularly in its early stages. This test determines whether a patient is anti-CCP2 positive or anti-CCP2 negative by comparing the measured ACPA levels against a defined cut-off value. In combination with the Rheumatoid Factor (RF) test, the anti-CCP2 test helps classify RA patients based on the presence of these specific serological markers, for both diagnosis and predicting disease progression. The anti-CCP2 test, while useful for diagnosing rheumatoid arthritis (RA), has certain limitations, particularly regarding its ability to predict disease severity. Although the presence of anti-cyclic citrullinated peptide antibodies (ACPAs) detected by the CCP2 test is strongly associated with the development of RA, it does not reliably indicate the severity or progression of the disease. While CCP2-positive individuals are at higher risk of developing RA and tend to experience more aggressive disease, the test does not distinguish between those who will develop severe forms of RA and those who may have a milder disease course. As such, being CCP2-positive does not necessarily correlate with a more benign or aggressive clinical outcome, and the test has limited value in predicting disease. Other biomarkers and clinical assessments are often needed to provide a more comprehensive prognosis regarding the severity of RA.

### Modifications

In one embodiment, the polypeptide further comprises a modification. Such modifications may serve various functions, including enhancing the stability, solubility, or biological activity of the polypeptide. The modification may also be employed to facilitate specific interactions or conjugations to other molecules or surfaces. Modifications may include chemical alterations or the addition of specific functional groups or molecules that allow the polypeptide to fulfil a specific role in diagnostic or therapeutic applications.

In one embodiment, the modification of the polypeptide comprises incorporation of non-natural amino acids or D-amino acids, acetylation, phosphorylation, cyclization, stapling, conjugation to a biomolecule, chelation, lipidation, N-terminal acetylation, C-terminal amidation, PEGylation, and/or glycosylation. In one embodiment, the modification of the polypeptide comprises acetylation, phosphorylation, cyclization, stapling, conjugation to a biomolecule, chelation, lipidation, or N-terminal acetylation.

In one embodiment, the polypeptide is cyclic. In some embodiments, the polypeptide is cyclized by a disulfide bridge between two cysteine residues. In some embodiments, the disulfide bridge is formed between the cysteine residue nearest the N-terminus and the cysteine residue nearest the C-terminus of the polypeptide. By cyclisation of the polypeptides, the epitopes recognised by ACPAs may be better presented to the immune system or to diagnostic detection systems, enhancing the likelihood of recognition by ACPAs. This is particularly advantageous for mimicking naturally occurring epitopes in citrullinated proteins, where the cyclic structure may more accurately reflect the conformations found *in vivo,* leading to improved recognition, specificity and sensitivity in diagnostic and/or prognostic assays. Further, cyclisation of polypeptides provides for increased resistance to proteolytic degradation, thus maintenance of structural integrity.

Cyclization of polypeptides can be achieved via techniques well-known in the art, such as solid-phase peptide synthesis, where cysteine residues are incorporated at desired positions in the sequence; oxidation to form the disulfide bridge, which results in the desired cyclic structure. The oxidation process can be achieved using agents well-known in the art, such as iodine or air oxidation under controlled conditions to ensure that the disulfide bridge forms specifically between the designated cysteine residues.

The cyclic peptides of the invention may be further modified by techniques such as substituting one or more amino acids near the cysteine residues to further influence the overall structure or flexibility of the cyclic polypeptide, altering the positioning of the cysteine residues so that the disulfide bridge is formed at alternative positions along the polypeptide sequence, which may modulate the conformation and potentially impact the binding affinity for ACPAs.

Cyclization of polypeptides can be achieved via techniques well-known in the art, such as solid-phase peptide synthesis, where cysteine residues are incorporated at desired positions in the sequence and subsequent oxidation to form the disulfide bridge; head-to-tail cyclization; lactam bridge formation between lysine and aspartic or glutamic acid residues; and thioether bond formation. These techniques provide a range of structural options for stabilizing the cyclic conformation. The oxidation process can be achieved using agents well-known in the art, such as iodine, dimethyl sulfoxide (DMSO), or air oxidation under controlled conditions to ensure that the disulfide bridge forms specifically between the designated cysteine residues. The cyclic peptides of the invention may be further modified by techniques such as substituting one or more amino acids near the cysteine residues to further influence the overall structure or flexibility of the cyclic polypeptide, altering the positioning of the cysteine residues so that the disulfide bridge is formed at alternative positions along the polypeptide sequence, which may modulate the conformation and potentially impact the binding affinity for ACPAs.

In one embodiment, the modification comprises conjugation to a moiety selected from the group comprising: biotin or derivative thereof, an amino acid, a polypeptide, a protein, a carbohydrate, a lipid, a fatty acid, a triglyceride, a steroid, polyethylene glycol (PEG), or a fluorophore. In some embodiments, the amino acid is an unnatural amino acid. In some embodiments the carbohydrate is selected from the group comprising a monosaccharide, disaccharide, or polysaccharide. In some embodiments the fatty acid is selected from the group comprising of saturated fatty acids and unsaturated fatty acids. In some embodiments the modification comprises conjugation to a chemical tag, a protein tag, a fluorescent tag and/or an isotopic tag.

In some embodiments the biotin moiety or derivative thereof is conjugated to the N-terminal end, the C-terminal end or an internal amino acid residue of the polypeptide. In some embodiments the biotin moiety or derivative thereof is conjugated to the N-terminal end of the polypeptide. In some embodiments the biotin moiety or a derivative thereof is conjugated to the polypeptide via a linking moiety. In some embodiments the linking moiety is selected from the group comprising aminohexanoic acid (Ahx), one or more amino acids, one or more peptides, maleimide, polyethylene glycol (PEG) or oligoethylene glycol. In some embodiments the linking moiety is aminohexanoic acid (Ahx).

In one embodiment, the polypeptide is selected from the group consisting of:
SEQ ID NO: 130: Biotin-Ahx-CSKAKFAG-Cit-NFRNPLACA
SEQ ID NO: 168: Biotin-Ahx-CVSPPVTV-Cit-GKEYPLGCA
SEQ ID NO: 163: Biotin-Ahx-CVRVFQAT-Cit-GKLSSKCA
SEQ ID NO: 179: Biotin-Ahx-CDFFTYHI-Cit-HGEVHCG, and
SEQ ID NO: 120: Biotin-Ahx-CEKGVPLY-Cit-HIADLAGCA.

In some embodiments, the polypeptide is SEQ ID NO: 130: Biotin-Ahx-CSKAKFAG-Cit-NFRNPLACA. In some embodiments, the polypeptide is SEQ ID NO: 168: Biotin-Ahx-CVSPPVTV-Cit-GKEYPLGCA. In some embodiments, the polypeptide is SEQ ID NO: 163: Biotin-Ahx-CVRVFQAT-Cit-GKLSSKCA. In some embodiments, the polypeptide is SEQ ID NO: 179: Biotin-Ahx-CDFFTYHI-Cit-HGEVHCG. In some embodiments, the polypeptide is SEQ ID NO: 120: Biotin-Ahx-CEKGVPLY-Cit-HIADLAGCA.

### Plurality of peptides

In one aspect, the invention provides for a plurality of polypeptides comprising at least two polypeptides as set forth in any of the above sections entitled `linear sequences',' cyclic sequences', 'binding to ACPAs' and 'modifications'.

In one embodiment the plurality of polypeptides comprises at least two polypeptides selected from the group comprising or consisting of SEQ ID NO: 1, 2, 3, 4, 5, 6, 7, 8, 9, 10, 11, 12, 13, 14, 15, 130, 168, 163, 179 and 120. In one embodiment the plurality of polypeptides comprises at least two polypeptides selected from the group comprising or consisting of SEQ ID NO: 1, 2, 3, 4, 5, 6 and/or 7, such as SEQ ID NO: 3-7.

In one embodiment the plurality of polypeptides comprises at least two polypeptides selected from the group comprising or consisting of SEQ ID NO: 8, 9, 10, 11, 12, 13, 14 and/or 15, such as SEQ ID NO: 11, 12, 13, 14 and/or 15. In one embodiment the plurality of polypeptides comprises the plurality of polypeptides have a net positive charge or a net balanced charge.

### Composition

In one aspect, the invention provides for a composition comprising at least one polypeptide according as set forth in any of the above sections entitled `linear sequences',' cyclic sequences', 'binding to ACPAs', 'modifications' and 'plurality of peptides'.

### Diagnosis/Prediction

In one aspect, the invention provides for a polypeptide for use in the diagnosis of autoimmune disease in a subject, wherein the polypeptide comprises or consists of a sequence selected from the group consisting of:
SEQ ID NO: 3: SKAKFAG-Cit-NFRNPLA
SEQ ID NO: 4: VSPPVTV-Cit-GKEYPLG
SEQ ID NO: 5: VRVFQAT-Cit-GKLSSK
SEQ ID NO: 6: DFFTYHI-Cit-HGEVHCG, and
SEQ ID NO: 7: EKGVPLY-Cit-HIADLAG.

In one aspect, the invention provides for a polypeptide for use in the prognosis of an autoimmune disease in an individual or identification of an individual at risk of developing an autoimmune disease, wherein the polypeptide comprises or consists of a sequence selected from the group consisting of:
SEQ ID NO: 3: SKAKFAG-Cit-NFRNPLA
SEQ ID NO: 4: VSPPVTV-Cit-GKEYPLG
SEQ ID NO: 5: VRVFQAT-Cit-GKLSSK
SEQ ID NO: 6: DFFTYHI-Cit-HGEVHCG, and
SEQ ID NO: 7: EKGVPLY-Cit-HIADLAG.

In one aspect, the invention provides for a polypeptide for use in the diagnosis of autoimmune disease in a subject, wherein the polypeptide is selected from the group consisting of:
SEQ ID NO: 11: CSKAKFAG-Cit-NFRNPLACA
SEQ ID NO: 12: CVSPPVTV-Cit-GKEYPLGCA
SEQ ID NO: 13: CVRVFQAT-Cit-GKLSSKCA
SEQ ID NO: 14: CDFFTYHI-Cit-HGEVHCG, and
SEQ ID NO: 15: CEKGVPLY-Cit-HIADLAGCA

In one aspect, the invention provides for a polypeptide for use in the prognosis of an autoimmune disease in an individual or identification of an individual at risk of developing an autoimmune disease, wherein the polypeptide is selected from the group consisting of:
SEQ ID NO: 11: CSKAKFAG-Cit-NFRNPLACA
SEQ ID NO: 12: CVSPPVTV-Cit-GKEYPLGCA
SEQ ID NO: 13: CVRVFQAT-Cit-GKLSSKCA
SEQ ID NO: 14: CDFFTYHI-Cit-HGEVHCG, and
SEQ ID NO: 15: CEKGVPLY-Cit-HIADLAGCA

In one aspect, the invention provides for a polypeptide for use in the diagnosis of autoimmune disease in a subject, wherein the polypeptide is selected from the group consisting of:
SEQ ID NO: 16130: Biotin-Ahx-CSKAKFAG-Cit-NFRNPLACA
SEQ ID NO: 16817: Biotin-Ahx-CVSPPVTV-Cit-GKEYPLGCA
SEQ ID NO: 16318: Biotin-Ahx-CVRVFQAT-Cit-GKLSSKCA
SEQ ID NO: 17919: Biotin-Ahx-CDFFTYHI-Cit-HGEVHCG, and
SEQ ID NO: 12020: Biotin-Ahx-CEKGVPLY-Cit-HIADLAGCA.

In one aspect, the invention provides for a polypeptide for use in the prognosis of an autoimmune disease in an individual or identification of an individual at risk of developing an autoimmune disease, wherein the polypeptide is selected from the group consisting of:
SEQ ID NO: 130: Biotin-Ahx-CSKAKFAG-Cit-NFRNPLACA
SEQ ID NO: 168: Biotin-Ahx-CVSPPVTV-Cit-GKEYPLGCA
SEQ ID NO: 163: Biotin-Ahx-CVRVFQAT-Cit-GKLSSKCA
SEQ ID NO: 179: Biotin-Ahx-CDFFTYHI-Cit-HGEVHCG, and
SEQ ID NO: 120: Biotin-Ahx-CEKGVPLY-Cit-HIADLAGCA.

In one aspect, the invention provides a polypeptide, plurality of polypeptides or composition as set forth in any of the above sections entitled `linear sequences',' cyclic sequences', 'binding to ACPAs', 'modifications' and 'plurality of peptides' for use in the diagnosis of an autoimmune disease in a subject. In some embodiments, the autoimmune disease is rheumatoid arthritis.

In one aspect, the invention provides a polypeptide, plurality of polypeptides or composition as set forth in any of the above sections entitled `linear sequences',' cyclic sequences', 'binding to ACPAs', 'modifications' and 'plurality of peptides' for use in the prognosis of an individual or identification of an individual at risk of developing an autoimmune disease. In some embodiments, the autoimmune disease is rheumatoid arthritis.

In some embodiments, the polypeptide is used to detect the presence of at least one ACPA in a sample obtained from the individual, and wherein binding of the polypeptide or plurality of polypeptides to the at least one ACPAs is indicative of a reduced risk of disease severity, progression and/or activity. In some embodiments, the reduced risk of disease severity, progression and/or activity in the individual is quantified by improved clinical outcomes, such as a decreased tender joint count (TJC), decreased swollen joint count (SJC), decreased erythrocyte sedimentation rate (ESR), decreased C-reactive protein levels (CRP), and/or a reduced DAS28 score.

In some embodiments, the reduced risk of disease severity, progression and/or activity in the individual is measured by improved clinical outcomes, such as a decreased tender joint count (TJC), decreased swollen joint count (SJC), decreased erythrocyte sedimentation rate (ESR), decreased C-reactive protein levels (CRP), and/or a reduced DAS28 score, as compared to an individual wherein the APCA present in the sample does not bind to the at least one polypeptide.

In some embodiments, the diagnosis of a subject, prognosis of an individual or identification of an individual at risk of developing an autoimmune disease, such as rheumatoid arthritis, is determined before the onset of clinical symptoms, such as at the most 12 years before the onset of clinical symptoms, such as at the most 11 years before the onset of clinical symptoms, such as at the most 10 years before the onset of clinical symptoms, such as at the most 9 years before the onset of clinical symptoms, such as at the most 8 years before the onset of clinical symptoms, such as at the most 7 years before the onset of clinical symptoms, such as at the most 6 years before the onset of clinical symptoms, such as at the most 5 years before the onset of clinical symptoms, such as at the most 4 years before the onset of clinical symptoms, such as at the most 3 years before the onset of clinical symptoms, such as at the most 2 years before the onset of clinical symptoms, such as at the most 1 year before the onset of clinical symptoms, such as at the most 6 months before the onset of clinical symptoms; or at the onset of clinical symptoms; or after the onset of clinical symptoms, such as at the most 1 year after onset of clinical symptoms, such as at the least 1 year after onset of clinical symptoms.

As used herein, the term "clinical symptoms" refers to observable and measurable clinical manifestations of a disease, such as rheumatoid arthritis (RA), in an individual and/or subject and/or patient, which may include both joint-related and systemic signs of disease. Clinical symptoms of RA typically encompass, but are not limited to, joint swelling (swollen joint count, SJC), joint tenderness (tender joint count, TJC), morning stiffness, and pain. These symptoms are key indicators of the inflammatory processes in RA and are routinely assessed in clinical practice. In addition to joint-related symptoms, systemic markers of inflammation such as elevated erythrocyte sedimentation rate (ESR) and increased C-reactive protein (CRP) levels are frequently measured as indicators of active inflammation. These markers are used to assess the severity of systemic inflammation and are well-known to those skilled in the art as reliable indicators of disease activity. ESR is measured by the rate at which red blood cells settle at the bottom of a test tube in one hour, with higher rates indicating increased inflammation. CRP, a protein produced by the liver, is measured using blood tests and reflects the level of inflammation in the body. Clinical symptoms are often quantified using composite scoring systems, such as the Disease Activity Score 28 (DAS28), which is a well-established tool for assessing RA disease severity. The DAS28 score combines the TJC, SJC, patient-reported global health, and inflammatory markers (ESR or CRP) to provide a comprehensive assessment of disease activity. It assesses 28 specific joints for tenderness and/or swelling. This score is widely used in clinical studies and treatment evaluations to monitor disease progression and response to therapy. Scores above 5.1 indicate high disease activity, while scores below 2.6 indicate remission. Methods for quantifying these clinical symptoms are standard in the field of RA research and are well known to those skilled in the art. Accordingly, in some embodiments, clinical symptoms may further include systemic inflammation markers such as elevated erythrocyte sedimentation rate (ESR) and increased C-reactive protein (CRP) levels, which are indicators of active inflammation. Additionally, clinical symptoms may be evaluated using composite scores such as the Disease Activity Score 28 (DAS28), which measures disease severity through parameters like the tender joint count (TJC) and swollen joint count (SJC), as well as inflammatory markers. In some embodiments, the sample is selected from the group comprising serum, plasma, saliva and synovial fluid.

### Methods

In one aspect, a method of diagnosing rheumatoid arthritis in a subject is provided, the method comprising exposing at least one polypeptide, plurality of polypeptides, or composition as set forth in in any of the above sections entitled `linear sequences',' cyclic sequences', 'binding to ACPAs', 'modifications', 'plurality of peptides', and 'composition', to a sample obtained from said subject, and detecting whether at least one antibody present in the sample binds to the at least one polypeptide. In one embodiment, the method comprises detecting whether at least one antibody present in a sample provided from the subject binds to at least one polypeptide as set forth in in any of the above sections entitled 'linear sequences',' cyclic sequences', 'binding to ACPAs', 'modifications', 'plurality of peptides', and 'composition'.

In one aspect, a method of prognosing rheumatoid arthritis in an individual, or identifying an individual at risk of developing rheumatoid arthritis is provided, the method comprising exposing at least one polypeptide, plurality of polypeptides, or composition as set forth in in any of the above sections entitled 'linear sequences',' cyclic sequences', 'binding to ACPAs', 'modifications', 'plurality of peptides', and 'composition' to a sample obtained from said individual, and detecting whether at least one antibody present in the sample binds to the at least one polypeptide. In one embodiment, the method comprises detecting whether at least one antibody present in a sample provided from the subject binds to at least one polypeptide as set forth in in any of the above sections entitled 'linear sequences', 'cyclic sequences', 'binding to ACPAs', 'modifications', 'plurality of peptides', and 'composition'.

In one aspect, a method of diagnosing rheumatoid arthritis in a subject, distinguishing between different types or stages of rheumatoid arthritis in a subject, prognosing rheumatoid arthritis in an individual or identifying an individual at risk of developing rheumatoid arthritis is provided, the method comprising detecting whether at least one antibody present in a sample provided from the subject or individual, binds to at least one polypeptide, plurality of polypeptides, or composition as set forth in in any of the above sections entitled `linear sequences', 'cyclic sequences', 'binding to ACPAs', 'modifications', 'plurality of peptides', and 'composition'.

In one embodiment, the method comprises detecting whether at least one antibody present in a sample provided from the subject or individual, binds to at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120. In one embodiment, the at least one polypeptide is immobilized on a surface.

In one aspect, a method of diagnosing rheumatoid arthritis in a subject is provided, the method comprising the steps of:
a) providing a sample obtained from the subject,
b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the at least one polypeptide is immobilized on a surface, and
c) detecting whether at least one antibody present in the sample binds to the at least one polypeptide.

In one aspect, a method of distinguishing between different types or stages of rheumatoid arthritis in a subject, prognosing rheumatoid arthritis in an individual or identifying an individual at risk of developing rheumatoid arthritis is provided, the method comprising the steps of:
a) providing a sample obtained from the subject or individual,
b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the at least one polypeptide is immobilized on a surface, and
c) detecting whether at least one antibody present in the sample binds to the at least one polypeptide.

In some embodiments, the method comprises the steps of:
a) providing a sample obtained from the subject or individual;
b) contacting the sample with at least one polypeptide comprising or consisting of a sequence selected from the group consisting of SEQ ID NO: 130, 168, 163, 179 and 120, wherein the at least one polypeptide is immobilized on a surface, and
c) detecting whether at least one antibody present in the sample binds to the at least one polypeptide.

In one aspect, a method for identifying an individual at risk of developing rheumatoid arthritis is provided, the method comprising the steps of:
a) providing a sample obtained from the individual;
b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120wherein the at least one polypeptide is immobilized on a surface, and
c) detecting whether at least one antibody present in the sample binds to the at least one polypeptide.

In some embodiments, the method comprises the steps of:
a) providing a sample obtained from the individual;
b) contacting the sample with at least one polypeptide comprising or consisting of a sequence selected from the group consisting of SEQ ID NO: 130, 168, 163, 179 and 120wherein the at least one polypeptide is immobilized on a surface, and
c) detecting whether at least one antibody present in the sample binds to the at least one polypeptide.

In one aspect, a method for the prognosis of rheumatoid arthritis in an individual is provided, the method comprising the steps of:
a) providing a sample obtained from the individual;
b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the at least one polypeptide is immobilized on a surface, and
c) detecting whether at least one antibody present in the sample binds to the at least one polypeptide, and
d) prognosing a reduced severity, progression and/or activity of disease in the individual when the at least one antibody present in the sample is detected to bind to the at least one polypeptide.

In some embodiments, the at least one antibody is as defined as set forth in the above-mentioned section entitled "binding to ACPAs". In some embodiments, the subject or individual is rheumatoid factor (RF) positive (seropositive). In some embodiments, the subject or individual is RF negative (seronegative).

In some embodiments, the reduced severity, progression and/or activity of disease in the individual is quantified by improved clinical outcomes, such as a decreased tender joint count (TJC), decreased swollen joint count (SJC), decreased erythrocyte sedimentation rate (ESR), decreased C-reactive protein levels (CRP), and/or a reduced DAS28 score. In some embodiments, the reduced severity, progression and/or activity of disease in the individual is measured by improved clinical outcomes, such as a decreased tender joint count (TJC), decreased swollen joint count (SJC), decreased erythrocyte sedimentation rate (ESR), decreased C-reactive protein levels (CRP), and/or a reduced DAS28 score, as compared to an individual wherein the antibody present in the sample does not bind to the at least one polypeptide.

In some embodiments, the at least one polypeptide is immobilized on a surface, wherein the surface is solid. In some embodiments, the surface comprises polypropylene, polystyrene, graphite, silica, glass, silicon, plastic, gold and/or microspheres. In some embodiments, the surface comprises microspheres, such as magnetic, polystyrene, glass, silica, polyacrylamide and/or latex beads. In some embodiments, the surface comprises avidin- and/or neutravidin-coated microspheres. In some embodiments, the at least one polypeptide is biotinylated and immobilized on a surface via a neutravidin-biotin interaction.

### Detection systems

In some embodiments, at least one antibody present in the sample binds to the at least one polypeptide comprises using a detection system selected from the group comprising a multiplex bead-based immunoassay system, flow cytometry, fluorescence-based detection, turbidimetry, radioisotope-based detection, and chemiluminescence-based detection. In some embodiments, the detection system is selected from the group comprising an enzyme-linked immunosorbent assay (ELISA), chemiluminescence immunoassay (CLIA), lateral flow immunoassay (LFIA), immunoturbidimetric assay, radioimmunoassay (RIA), electrochemiluminescence (ECL) and electrochemiluminescence immunoassay (ECLIA).

In some embodiments, the detecting whether the at least one antibody present in the sample binds to said at least one polypeptide comprises use of a detection reagent selected from the group consisting of enzyme-conjugated antibodies, fluorescence-conjugated antibodies, gold nanoparticles or luminescent probes. In some embodiments, the detection reagent is selected from the group comprising horseradish peroxidase (HRP), alkaline phosphatase (AP), R-phycoerythrin (R-PE), or chemiluminescent substrates.

In some embodiments, detecting whether the at least one antibody present in the sample binds to said at least one polypeptide is quantified by measuring the signal intensity of the detection reagent. In some embodiments, detecting whether the at least one antibody present in the sample binds to said at least one polypeptide is quantified by measuring the signal intensity of the detection reagent, wherein the detection reagent is selected from the group comprising horseradish peroxidase (HRP), alkaline phosphatase (AP), R-phycoerythrin (R-PE), or chemiluminescent substrates.

In some embodiments, the method further comprises;
a. the at least one polypeptide is immobilized on a surface comprising a microplate coated with neutravidin, and
b. detecting whether the at least one antibody present in the sample binds to said at least one polypeptide comprises using a detection system, wherein the detection system is an enzyme-linked immunosorbent assay (ELISA).

In some embodiments, the detection system allows for simultaneous detection of the at least one antibody binding to a panel comprising at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120. In some embodiments, the detection system allows for simultaneous detection of the at least one antibody binding to a panel comprising at least one polypeptide comprising or consisting of a sequence selected from the group consisting of SEQ ID NO: 130, 168, 163, 179 and 120.

In some embodiments, the subject and/or individual is pre-symptomatic or symptomatic. As used herein, the term "symptomatic" refers to an individual who exhibits clinical signs or symptoms associated with rheumatoid arthritis (RA). These symptoms may include, but are not limited to, joint pain, swelling, tenderness, stiffness, reduced range of motion, and other manifestations of inflammation and joint damage commonly observed in RA patients. The symptomatic state indicates the presence of observable and measurable clinical manifestations of the disease.

As used herein, the term "pre-symptomatic" refers to an individual who does not yet exhibit clinical signs or symptoms of RA, but may possess biological markers or risk factors, such as the presence of specific autoantibodies (e.g., anti-citrullinated protein antibodies (ACPAs)) or genetic predispositions, indicating a heightened likelihood of developing RA in the future. Pre-symptomatic individuals may have detectable immune reactivity associated with RA without showing physical symptoms, thus allowing for early identification and potential intervention before clinical onset.

As used herein, the term "onset of clinical symptoms" refers to the point in time at which an individual begins to exhibit observable and measurable signs or symptoms associated with RA. These symptoms may include, but are not limited to, joint pain, swelling, stiffness, reduced range of motion, and other indications of inflammation and joint dysfunction that are characteristic of RA. The onset of clinical symptoms marks the transition from a pre-symptomatic or latent stage to an active, symptomatic phase of the disease, where clinical diagnosis is typically made based on the manifestation of such symptoms.

As used herein, the term "early rheumatoid arthritis" or "early RA" refers to the stage of rheumatoid arthritis (RA) that occurs within 0-1 years following the onset of clinical symptoms and/or at the time of RA diagnosis and/or 0-1 years following the diagnosis of RA. This phase is characterised by the initial presentation of RA-related symptoms such as joint pain, swelling, stiffness, and inflammation. Early RA is typically the period during which early interventions and treatments are initiated to potentially slow disease progression, manage symptoms, and prevent long-term joint damage.

In some embodiments, the subject and/or individual is identified before the onset of clinical symptoms, such as at the most 12 years before the onset of clinical symptoms, such as at the most 11 years before the onset of clinical symptoms, such as at the most 10 years before the onset of clinical symptoms, such as at the most 9 years before the onset of clinical symptoms, such as at the most 8 years before the onset of clinical symptoms, such as at the most 7 years before the onset of clinical symptoms, such as at the most 6 years before the onset of clinical symptoms, such as at the most 5 years before the onset of clinical symptoms, such as at the most 4 years before the onset of clinical symptoms, such as at the most 3 years before the onset of clinical symptoms, such as at the most 2 years before the onset of clinical symptoms, such as at the most 1 year before the onset of clinical symptoms, such as at the most 6 months before the onset of clinical symptoms; or at the onset of clinical symptoms; or after the onset of clinical symptoms, such as at the most 1 year after onset of clinical symptoms, such as at the least 1 year after onset of clinical symptoms.

In some embodiments, the diagnosis in a subject, distinguishing between different types or stages of rheumatoid arthritis in a subject, or prognosis of rheumatoid arthritis in an individual is determined before the onset of clinical symptoms, such as at the most 12 years before the onset of clinical symptoms, such as at the most 11 years before the onset of clinical symptoms, such as at the most 10 years before the onset of clinical symptoms, such as at the most 9 years before the onset of clinical symptoms, such as at the most 8 years before the onset of clinical symptoms, such as at the most 7 years before the onset of clinical symptoms, such as at the most 6 years before the onset of clinical symptoms, such as at the most 5 years before the onset of clinical symptoms, such as at the most 4 years before the onset of clinical symptoms, such as at the most 3 years before the onset of clinical symptoms, such as at the most 2 years before the onset of clinical symptoms, such as at the most 1 year before the onset of clinical symptoms, such as at the most 6 months before the onset of clinical symptoms; or at the onset of clinical symptoms; or after the onset of clinical symptoms, such as at the most 1 year after onset of clinical symptoms, such as at the least 1 year after onset of clinical symptoms.

As used herein, the term "clinical parameter" refers to any measurable factor, data, or observation related to the health status or condition of an individual, specifically in the context of RA. These parameters may include, but are not limited to, clinical symptoms of RA (such as joint pain, swelling, stiffness, or tenderness), serological markers (such as RF) or anti-citrullinated protein antibodies (ACPA)), imaging results (e.g., X-ray, ultrasound, or Magnetic Resonance Imaging (MRI)), demographic factors (such as age and gender), medical history (such as family history of autoimmune diseases or personal history of joint trauma), genetic markers (such as HLA-DRB1, PTPN22, or STAT4), environmental influences (e.g., smoking or infections), and patient-reported outcomes (including pain levels, physical function, and quality of life). In some embodiments, the method further comprises obtaining one or more clinical parameter. In some embodiments, the one or more clinical parameter is utilised in combination with detecting whether at least one antibody present in the sample binds to the at least one polypeptide.

### Companion diagnostics

In one aspect, a method for selecting an individual having RA is provided, the method comprising:
a) providing a sample obtained from an individual;
b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the polypeptide is immobilized on a surface,
c) detecting whether at least one antibody present in the sample binds to the at least one polypeptide,
d) selecting the individual having RA following detection of binding of the at least one antibody present in the sample to the at least one polypeptide.

In one aspect, a method for selecting an individual at risk of developing RA is provided, the method comprising;
a) providing a sample obtained from an individual, wherein the individual does not exhibit clinical symptoms of RA,
b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the polypeptide is immobilized on a surface,
c) detecting whether at least one antibody present in the sample binds to the at least one polypeptide,
d) selecting the individual having RA following detection of binding of the at least one antibody present in the sample to the at least one polypeptide.

In some embodiments, the method further comprises administration of at least one therapeutic agent. In some embodiments, the at least one polypeptide comprising or consisting of a sequence selected from the group consisting of SEQ ID NO: 130, 168, 163, 179 and 120. In some embodiments, the at least one therapeutic agent selected from the group comprising anti-inflammatory and immunosuppressive therapeutics, conventional Disease-Modifying Anti-Rheumatic Drugs (cDMARDs), biologic DMARDs (bDMARDs), Targeted Synthetic DMARDs (tsDMARDs); agents which modulate of regulatory T cells (Tregs), regulatory B cells (Bregs), suppressor B cells (Bsups), or autoreactive T and/or B cells; Stem Cell Therapy, analgesics, and/or Peptidyl arginine deiminase 4 (PAD4) inhibitors. In some embodiments, the anti-inflammatory and immunosuppressive therapeutics comprise non-steroidal anti-inflammatory Agents (NSAIDs), such as naproxen, indomethacin and/or COX-2 inhibitors, and/or corticosteroids. In some embodiments, the conventional Disease-Modifying Anti-Rheumatic Drugs (cDMARDs) comprise methotrexate, sulfasalazine, hydroxychloroquine, and/or leflunomide. In some embodiments, the biologic DMARDs (bDMARDs), comprise TNF Inhibitors, such as adalimumab, etanercept, infliximab, certolizumab pegol, and golimumab; IL-6 Inhibitors, such as tocilizumab or sarilumab; B-cell depleting antibodies, such as rituximab; T-cell Co-stimulation Modulators, such as abatacept; Interleukin-1 receptor antagonists (IL-1Ra), such as anakinra; otilimab, bispecific or trispecific antibodies which modulate immune response and/or inflammatory processes, and/or novel monoclonal antibodies, such as ACPAs. In some embodiments, the targeted synthetic DMARDs (tsDMARDs) comprise Janus Kinase (JAK) inhibitors, such as tofacitinib, baricitinib, upadacitinib, filgotinib, and peficitinib; and/or Bruton's Tyrosine Kinase (BTK) inhibitors, such as fenebrutinib.

In some embodiments, the agents which modulate of regulatory T cells (Tregs), regulatory B cells (Bregs), or autoreactive T and/or B cells comprise agents which activate regulatory T cells or B cells, tolerogenic vaccines, and/or cell therapies for the modulation or expansion of the individual's regulatory cells. In some embodiments, stem cell therapy comprises use of the individual's own mesenchymal stem cells (MSCs) for synovial MSC therapy.

In some embodiments, the selection of the individual is determined based on the binding and/or binding affinity of the at least one antibody to the at least one polypeptide, wherein the binding and/or binding affinity of said antibody informs the selection of combination therapies of two or more therapeutic agents. In some embodiments, the sample is selected from the group comprising serum, plasma, saliva and synovial fluid.

### Kit

In one aspect, a kit of parts is provided comprising;
a) at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120 as set forth in any of the above sections entitled `linear sequences', 'cyclic sequences', 'binding to ACPAs', 'modifications', 'plurality of peptides', and 'composition', wherein the at least one polypeptide is immobilized on a surface; and
b) at least one detection reagent selected from the group comprising enzyme-conjugated antibodies, fluorescence-conjugated antibodies, or luminescent probes for detecting antibody binding to the at least one polypeptide.

In some embodiments, the kit of parts comprises at least one polypeptide comprising or consisting of a sequence selected from the group consisting of SEQ ID NO: 130, 168, 163, 179 and 120. In some embodiments, the detection reagent comprises horseradish peroxidase (HRP), alkaline phosphatase (AP), fluorescent labels such as R-phycoerythrin (R-PE), radioisotopes, electrochemical labels, gold nanoparticles, quantum dots, bioluminescent enzymes or chemiluminescent substrates. In some embodiments, the kit further comprises a secondary reagent for enhancing detection of antibody binding, wherein the secondary reagent is selected from the group comprising tyramide signal amplification (TSA) reagents, biotinylated antibodies, or streptavidinconjugated probes. In some embodiments, the surface comprises a material selected from the group comprising polypropylene, polystyrene, graphite, silica, glass, silicon, plastic, gold, and/or microspheres. In some embodiments, the kit further comprises at least one antibody as set forth in the of the above section entitled 'binding to ACPAs'.

### Items

### Linear sequences

1. A polypeptide comprising or consisting of the sequence
   X₁ - X₂ - X₃ - X₄ - X₅ - X₆ - X₇ - Cit - X_{8 -} X₉ - X₁₀ - X₁₁ - X₁₂ - X₁₃ - X₁₄ (SEQ ID NO: 1)
   X₁ is Serine (S), Valine (V), Aspartic Acid (D) or Glutamic Acid (E),
   X₂ is Lysine (K), Serine (S), Arginine (R) or Phenylalanine (F),
   X₃ is Alanine (A), Proline (P), Valine (V), Phenylalanine (F) or Glycine (G),
   X₄ is Lysine (K), Proline (P), Phenylalanine (F), Threonine (T) or Valine (V),
   X₅ is Phenylalanine (F), Valine (V), Glutamine (Q), Tyrosine (Y) or Proline (P),
   X₆ is Alanine (A), Threonine (T), Histidine (H) or Leucine (L),
   X₇ is Glycine (G), Valine (V), Threonine (T), Isoleucine (I) or Tyrosine (Y),
   X₈ is Asparagine (N), Glycine (G) or Histidine (H),
   X₉ is Phenylalanine (F), Lysine (K), Glycine (G) or Isoleucine (I),
   X₁₀ is Arginine (R), Glutamic Acid (E), Alanine (A) or absent,
   X₁₁ is Asparagine (N), Tyrosine (Y), Leucine (L), Valine (V) or Aspartic Acid (D),
   X₁₂ is Proline (P), Serine (S), Histidine (H) or Leucine (L),
   is Leucine (L), Serine (S), or Alanine (A),
   X₁₄ is Alanine (A), Glycine (G) or Lysine (K).
2. The polypeptide according to any one of the preceding items, wherein the polypeptide comprises or consists of the sequence
   X₁ - X₂ - X₃ - X₄ - X₅ - X₆ - X₇ - Cit - X_{8 -} X₉ - X₁₀ - X₁₁ - X₁₂ - X₁₃ - X₁₄ (SEQ ID NO: 1)
   X₁ is Serine (S), Valine (V) or Aspartic Acid (D),
   X₂ is Lysine (K), Serine (S), Arginine (R) or Phenylalanine (F),
   X₃ is Alanine (A), Proline (P), Valine (V) or Phenylalanine (F),
   X₄ is Lysine (K), Proline (P), Phenylalanine (F) or Threonine (T),
   X₅ is Phenylalanine (F), Valine (V), Glutamine (Q) or Tyrosine (Y),
   X₆ is Alanine (A), Threonine (T) or Histidine (H),
   X₇ is Glycine (G), Valine (V), Threonine (T) or Isoleucine (I),
   X₈ is Asparagine (N), Glycine (G) or Histidine (H),
   X₉ is Phenylalanine (F), Lysine (K) or Glycine (G),
   X₁₀ is Arginine (R), Glutamic Acid (E) or absent,
   X₁₁ is Asparagine (N), Tyrosine (Y), Leucine (L) or Valine (V),
   X₁₂ is Proline (P), Serine (S) or Histidine (H),
   is Leucine (L), or Serine (S),
   X₁₄ is Alanine (A), Glycine (G) or Cysteine (C).
3. The polypeptide according to any one of the preceding items, wherein the polypeptide comprises or consists of the sequence
   X₁ - X₂ - X₃ - X₄ - X₅ - X₆ - X₇ - Cit - X_{8 -} X₉ - X₁₀ - X₁₁ - X₁₂ - X₁₃ - X₁₄ (SEQ ID NO: 1)
   X₁ is Serine (S) or Valine (V),
   X₂ is Lysine (K), Serine (S) or Arginine (R),
   X₃ is Alanine (A), Proline (P) or Valine (V),
   X₄ is Lysine (K), Proline (P) or Phenylalanine (F),
   X₅ is Phenylalanine (F), Valine (V) or Glutamine (Q),
   X₆ is Alanine (A) or Threonine (T),
   X₇ is Glycine (G), Valine (V) or Threonine (T),
   X₈ is Asparagine (N) or Glycine (G),
   X₉ is Phenylalanine (F) or Lysine (K),
   X₁₀ is Arginine (R) or Glutamic Acid (E) or absent,
   X₁₁ is Asparagine (N), Tyrosine (Y) or Leucine (L),
   X₁₂ is Proline (P) or Serine (S),
   X₁₃ is Leucine (L) or Serine (S),
   X₁₄ is Alanine (A), Glycine (G) or Lysine (K).
4. The polypeptide according to any one of the preceding items, wherein the polypeptide comprises or consists of the sequence
   X₁ - X₂ - X₃ - X₄ - X₅ - X₆ - X₇ - Cit - X_{8 -} X₉ - X₁₀ - X₁₁ - P - L - X₁₄ (SEQ ID NO: 2)
   X₁ is Serine (S) or Valine (V),
   X₂ is Lysine (K) or Serine (S),
   X₃ is Alanine (A) or Proline (P),
   X₄ is Lysine (K) or Proline (P),
   X₅ is Phenylalanine (F) or Valine (V),
   X₆ is Alanine (A) or Threonine (T),
   X₇ is Glycine (G) or Valine (V),
   X₈ is Asparagine (N) or Glycine (G),
   X₉ is Phenylalanine (F) or Lysine (K),
   X₁₀ is Arginine (R) or Glutamic Acid (E),
   X₁₁ is Asparagine (N) or Tyrosine (Y),
   X₁₂ is Alanine (A) or Glycine (G).
5. The polypeptide according to any one of the preceding items, wherein the polypeptide comprises or consists of the sequence:

   A - Cit - B

   wherein A comprises an amino acid sequence consisting of 8 contiguous amino acid residues selected from the group consisting of SKAKFAG (SEQ ID NO; 21); VSPPVTV (SEQ ID NO; 22); VRVFQAT (SEQ ID NO; 23); DFFTYHI (SEQ ID NO; 24); EKGVPLY (SEQ ID NO; 25); and
   wherein B comprises an amino acid sequence consisting of between 7 and 9 contiguous amino acid residues selected from the group consisting of NFRNPLA (SEQ ID NO; 26); GKEYPLG (SEQ ID NO; 27); GKLSSK (SEQ ID NO; 28); HGEVHCG (SEQ ID NO; 29); HIADLAG (SEQ ID NO; 30).
6. The polypeptide according to any one of the preceding items, wherein the polypeptide is selected from the group consisting of:
   SEQ ID NO: 3: SKAKFAG-Cit-NFRNPLA
   SEQ ID NO: 4: VSPPVTV-Cit-GKEYPLG
   SEQ ID NO: 5: VRVFQAT-Cit-GKLSSK
   SEQ ID NO: 6: DFFTYHI-Cit-HGEVHCG, and
   SEQ ID NO: 7: EKGVPLY-Cit-HIADLAG
7. The polypeptide according to any one of the preceding items, wherein the polypeptide is SEQ ID NO: 7: EKGVPLY-Cit-HIADLAG.
8. The polypeptide according to any one of the preceding items, wherein the polypeptide is selected from the group consisting of:
   SEQ ID NO: 3: SKAKFAG-Cit-NFRNPLA
   SEQ ID NO: 4: VSPPVTV-Cit-GKEYPLG
   SEQ ID NO: 5: VRVFQAT-Cit-GKLSSK, and
   SEQ ID NO: 6: DFFTYHI-Cit-HGEVHCG.
9. The polypeptide according to any one of the preceding items, wherein the polypeptide is SEQ ID NO: 3: SKAKFAG-Cit-NFRNPLA.
10. The polypeptide according to any one of the preceding items, wherein the polypeptide is SEQ ID NO: 4: VSPPVTV-Cit-GKEYPLG.
11. The polypeptide according to any one of the preceding items, wherein the polypeptide is SEQ ID NO: 5: VRVFQAT-Cit-GKLSSK.
12. The polypeptide according to any one of the preceding items, wherein the polypeptide is SEQ ID NO: 6: DFFTYHI-Cit-HGEVHCG.

### Cyclic sequences

13. A polypeptide comprising or consisting of the sequence
   C - X₁ - X₂ - X₃ - X₄ - X₅ - X₆ - X₇ - Cit - X_{8 -} X₉ - X₁₀ - X₁₁ - X₁₂ - X₁₃ - X₁₄ - X₁₅ - X₁₆ (SEQ ID NO: 8)
   X₁ is Serine (S), Valine (V), Aspartic Acid (D) or Glutamic Acid (E),
   X₂ is Lysine (K), Serine (S), Arginine (R) or Phenylalanine (F),
   X₃ is Alanine (A), Proline (P), Valine (V), Phenylalanine (F) or Glycine (G),
   X₄ is Lysine (K), Proline (P), Phenylalanine (F), Threonine (T) or Valine (V),
   X₅ is Phenylalanine (F), Valine (V), Glutamine (Q), Tyrosine (Y) or Proline (P),
   X₆ is Alanine (A), Threonine (T), Histidine (H) or Leucine (L),
   X₇ is Glycine (G), Valine (V), Threonine (T), Isoleucine (I) or Tyrosine (Y),
   X₈ is Asparagine (N), Glycine (G) or Histidine (H),
   X₉ is Phenylalanine (F), Lysine (K), Glycine (G) or Isoleucine (I),
   X₁₀ is Arginine (R), Glutamic Acid (E), Alanine (A) or absent,
   X₁₁ is Asparagine (N), Tyrosine (Y), Leucine (L), Valine (V) or Aspartic Acid (D),
   X₁₂ is Proline (P), Serine (S), Histidine (H) or Leucine (L),
   is Leucine (L), Serine (S), Cysteine (C) or Alanine (A),
   X₁₄ is Alanine (A), Glycine (G) or Lysine (K),
   X₁₅ is absent or Cysteine (C),
   X₁₆ is absent or Alanine (A).
14. The polypeptide according to any one of the preceding items, wherein the polypeptide comprises or consists of the sequence
   C - X₁ - X₂ - X₃ - X₄ - X₅ - X₆ - X₇ - Cit - X_{8 -} X₉ - X₁₀ - X₁₁ - X₁₂ - X₁₃ - X₁₄ - X₁₅ - X₁₆ (SEQ ID NO: 8)
   X₁ is Serine (S), Valine (V) or Aspartic Acid (D),
   X₂ is Lysine (K), Serine (S), Arginine (R) or Phenylalanine (F),
   X₃ is Alanine (A), Proline (P), Valine (V) or Phenylalanine (F),
   X₄ is Lysine (K), Proline (P), Phenylalanine (F) or Threonine (T),
   X₅ is Phenylalanine (F), Valine (V), Glutamine (Q) or Tyrosine (Y),
   X₆ is Alanine (A), Threonine (T) or Histidine (H),
   X₇ is Glycine (G), Valine (V), Threonine (T) or Isoleucine (I),
   X₈ is Asparagine (N), Glycine (G) or Histidine (H),
   X₉ is Phenylalanine (F), Lysine (K) or Glycine (G),
   X₁₀ is Arginine (R), Glutamic Acid (E) or absent,
   X₁₁ is Asparagine (N), Tyrosine (Y), Leucine (L) or Valine (V),
   X₁₂ is Proline (P), Serine (S) or Histidine (H),
   is Leucine (L), Serine (S) or Cysteine (C),
   X₁₄ is Alanine (A), Glycine (G) or Cysteine (C),
   X₁₅ is absent or Cysteine (C),
   X₁₆ is absent or Alanine (A).
15. The polypeptide according to any one of the preceding items, wherein the polypeptide comprises or consists of the sequence
   C - X₁ - X₂ - X₃ - X₄ - X₅ - X₆ - X₇ - Cit - X_{8 -} X₉ - X₁₀ - X₁₁ - X₁₂ - X₁₃ - X₁₄ - C- A (SEQ ID NO: 9)
   X₁ is Serine (S) or Valine (V),
   X₂ is Lysine (K), Serine (S) or Arginine (R),
   X₃ is Alanine (A), Proline (P) or Valine (V),
   X₄ is Lysine (K), Proline (P) or Phenylalanine (F),
   X₅ is Phenylalanine (F), Valine (V) or Glutamine (Q),
   X₆ is Alanine (A) or Threonine (T),
   X₇ is Glycine (G), Valine (V) or Threonine (T),
   X₈ is Asparagine (N) or Glycine (G),
   X₉ is Phenylalanine (F) or Lysine (K),
   X₁₀ is Arginine (R) or Glutamic Acid (E) or absent,
   X₁₁ is Asparagine (N), Tyrosine (Y) or Leucine (L),
   X₁₂ is Proline (P) or Serine (S),
   is Leucine (L) or Serine (S),
   X₁₄ is Alanine (A), Glycine (G) or Lysine (K).
16. The polypeptide according to any one of the preceding items, wherein the polypeptide comprises or consists of the sequence
   C - X₁ - X₂ - X₃ - X₄ - X₅ - X₆ - X₇ - Cit - X_{8 -} X₉ - X₁₀ - X₁₁ - P - L- X₁₂ - C - A (SEQ ID NO: 10)
   X₁ is Serine (S) or Valine (V),
   X₂ is Lysine (K) or Serine (S),
   X₃ is Alanine (A) or Proline (P),
   X₄ is Lysine (K) or Proline (P),
   X₅ is Phenylalanine (F) or Valine (V),
   X₆ is Alanine (A) or Threonine (T),
   X₇ is Glycine (G) or Valine (V),
   X₈ is Asparagine (N) or Glycine (G),
   X₉ is Phenylalanine (F) or Lysine (K),
   X₁₀ is Arginine (R) or Glutamic Acid (E),
   X₁₁ is Asparagine (N) or Tyrosine (Y),
   X₁₂ is Alanine (A) or Glycine (G).
17. The polypeptide according to any one of the preceding items, wherein the polypeptide comprises or consists of the sequence:

   A - Cit - B

   wherein A comprises an amino acid sequence consisting of 8 contiguous amino acid residues selected from the group consisting of CSKAKFAG (SEQ ID NO; 31); CVSPPVTV (SEQ ID NO; 32); CVRVFQAT (SEQ ID NO; 33); CDFFTYHI (SEQ ID NO; 34); CEKGVPLY (SEQ ID NO; 35); and
   wherein B comprises an amino acid sequence consisting of between 7 and 9 contiguous amino acid residues selected from the group consisting of NFRNPLACA (SEQ ID NO; 36); GKEYPLGCA (SEQ ID NO; 37); GKLSSKCA (SEQ ID NO; 38); HGEVHCG (SEQ ID NO; 39); HIADLAGCA (SEQ ID NO; 40).
18. The polypeptide according to any one of the preceding items, wherein the polypeptide is selected from the group consisting of:
   SEQ ID NO: 11: CSKAKFAG-Cit-NFRNPLACA
   SEQ ID NO: 12: CVSPPVTV-Cit-GKEYPLGCA
   SEQ ID NO: 13: CVRVFQAT-Cit-GKLSSKCA
   SEQ ID NO: 14: CDFFTYHI-Cit-HGEVHCG, and
   SEQ ID NO: 15: CEKGVPLY-Cit-HIADLAGCA
19. The polypeptide according to any one of the preceding items, wherein the polypeptide is SEQ ID NO: 15: CEKGVPLY-Cit-HIADLAGCA.
20. The polypeptide according to any one of the preceding items, wherein the polypeptide is selected from the group consisting of:
   SEQ ID NO: 11: CSKAKFAG-Cit-NFRNPLACA
   SEQ ID NO: 12: CVSPPVTV-Cit-GKEYPLGCA
   SEQ ID NO: 13: CVRVFQAT-Cit-GKLSSKCA, and
   SEQ ID NO: 14: CDFFTYHI-Cit-HGEVHCG.
21. The polypeptide according to any one of the preceding items, wherein the polypeptide is SEQ ID NO: 11: CSKAKFAG-Cit-NFRNPLACA.
22. The polypeptide according to any one of the preceding items, wherein the polypeptide is SEQ ID NO: 12: CVSPPVTV-Cit-GKEYPLGCA.
23. The polypeptide according to any one of the preceding items, wherein the polypeptide is SEQ ID NO: 13: CVRVFQAT-Cit-GKLSSKCA.
24. The polypeptide according to any one of the preceding items, wherein the polypeptide is SEQ ID NO: 14: CDFFTYHI-Cit-HGEVHCG.
25. The polypeptide according to any one of the preceding items, wherein the polypeptide consists of between 12 to 20 amino acid residues.
26. The polypeptide according to any one of the preceding items, wherein the polypeptide consists of between 12 to 20 amino acid residues, such as between 13 to 20 amino acid residues, such as between 14 to 20 amino acid residues, such as between 15 to 20 amino acid residues, such as between 13 to 19 amino acid residues, such as between 13 to 18 amino acid residues, such as between 13 to 18 amino acid residues, such as between 14 to 18 amino acid residues, such as between 14 to 17 amino acid residues, such as between 14 to 16 amino acid residues, such as between 14 to 15 amino acid residues.
27. The polypeptide according to any one of the preceding items, wherein the polypeptide consists of between 14 to 18 amino acid residues.
28. The polypeptide according to any one of the preceding items, wherein the polypeptide consists of between 14 to 15 amino acid residues.
29. The polypeptide according to any one of the preceding items, wherein the polypeptide has a net positive charge.
30. The polypeptide according to item 29, wherein the polypeptide is selected from the group consisting of:
   SEQ ID NO: 3: SKAKFAG-Cit-NFRNPLA, and
   SEQ ID NO: 5: VRVFQAT-Cit-GKLSSK.
31. The polypeptide according to any one of the preceding items, wherein the polypeptide has a net neutral charge.
32. The polypeptide according to item 31, wherein the polypeptide is selected from the group consisting of:
   SEQ ID NO: 4: VSPPVTV-Cit-GKEYPLG
   SEQ ID NO: 6: DFFTYHI-Cit-HGEVHCG, and
   SEQ ID NO: 7: EKGVPLY-Cit-HIADLAG.
33. The polypeptide according to any one of the preceding items, wherein the polypeptide is hydrophobic.
34. A polypeptide comprising or consisting of a sequence selected from the group consisting of SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123, SEQ ID NO: 124, SEQ ID NO: 125, SEQ ID NO: 126, SEQ ID NO: 127, SEQ ID NO: 128, SEQ ID NO: 129, SEQ ID NO: 130, SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, SEQ ID NO: 162, SEQ ID NO: 163, SEQ ID NO: 164, SEQ ID NO: 165, SEQ ID NO: 166, SEQ ID NO: 167, SEQ ID NO: 168, SEQ ID NO: 169, SEQ ID NO: 170, SEQ ID NO: 171, SEQ ID NO: 172, SEQ ID NO: 173, SEQ ID NO: 174, SEQ ID NO: 175, SEQ ID NO: 176, SEQ ID NO: 177, SEQ ID NO: 178, SEQ ID NO: 179, SEQ ID NO: 180, and SEQ ID NO: 181.

### Binding to ACPAs

35. The polypeptide according to any one of the preceding items, wherein the polypeptide binds to at least one anti-citrullinated protein antibody (ACPA) or fragment thereof.
36. The polypeptide according to any one of the preceding items, wherein the at least one anti-citrullinated protein antibody or fragment thereof comprises a variable heavy chain (VH) domain and a variable light chain (VL) domain, and wherein
   a) the VH domain comprises an amino acid sequence that includes complementarity determining regions (CDRs);
      i. a HCDR1 sequence comprising an amino acid sequence as set forth in SEQ ID NO: 45; a HCDR2 sequence comprising an amino acid sequence as set forth in SEQ ID NO: 46; and a HCDR3 sequence comprising an amino acid sequence as set forth in SEQ ID NO: 43 or 47, or
      ii. a HCDR1 sequence comprising an amino acid sequence as set forth in SEQ ID NO: 61; a HCDR2 sequence comprising an amino acid sequence as set forth in SEQ ID NO: 62; and a HCDR3 sequence comprising an amino acid sequence as set forth in SEQ ID NO: 63, or
      iii. a HCDR1 sequence comprising an amino acid sequence as set forth in SEQ ID NO: 70; a HCDR2 sequence comprising an amino acid sequence as set forth in SEQ ID NO: 71; and a HCDR3 sequence comprising an amino acid sequence as set forth in SEQ ID NO: 72, or
      iv. a HCDR1 sequence comprising an amino acid sequence as set forth in SEQ ID NO: 90; a HCDR2 sequence comprising an amino acid sequence as set forth in SEQ ID NO: 91; and a HCDR3 sequence comprising an amino acid sequence as set forth in SEQ ID NO: 92, and
   b) the VL domain comprises an amino acid sequence that includes complementarity determining regions (CDRs);
      i. a LCDR1 sequence comprising an amino acid sequence as set forth in SEQ ID NO: 48; a LCDR2 sequence comprising an amino acid sequence as set forth in SEQ ID NO: 49; and a LCDR3 sequence comprising an amino acid sequence as set forth in SEQ ID NO: 44 or 50, or
      ii. a LCDR1 sequence comprising an amino acid sequence as set forth in SEQ ID NO: 53; a LCDR2 sequence comprising an amino acid sequence as set forth in SEQ ID NO: 54; and a LCDR3 sequence comprising an amino acid sequence as set forth in SEQ ID NO: 55, or
      iii. a LCDR1 sequence comprising an amino acid sequence as set forth in SEQ ID NO: 79; a LCDR2 sequence comprising an amino acid sequence as set forth in SEQ ID NO: 80; and a LCDR3 sequence comprising an amino acid sequence as set forth in SEQ ID NO: 81, or
      iv. a LCDR1 sequence comprising an amino acid sequence as set forth in SEQ ID NO: 93; a LCDR2 sequence comprising an amino acid sequence as set forth in SEQ ID NO: 94; and a LCDR3 sequence comprising an amino acid sequence as set forth in SEQ ID NO: 95.
37. The polypeptide according to any one of the preceding items, wherein the at least one anti-citrullinated protein antibody or fragment thereof comprises;
   a) a variable heavy chain (VH) domain comprising an amino acid sequence selected from SEQ ID NO: 52, SEQ ID NO: 69, SEQ ID NO: 78 or SEQ ID NO: 88; and
   b) a variable light chain (VL) domain comprising an amino acid sequence selected from SEQ ID NO: 51, SEQ ID NO: 60, SEQ ID NO: 87 or SEQ ID NO: 89.
38. The polypeptide according to any one of the preceding items, wherein the at least one anti-citrullinated protein antibody or fragment thereof comprises a variable heavy chain (VH) domain and a variable light chain (VL) domain, and wherein
   a) the VH domain comprises an amino acid sequence that includes complementarity determining regions (CDRs):
      i. a HCDR1 sequence comprising an amino acid sequence as set forth in SEQ ID NO: 61; a HCDR2 sequence comprising an amino acid sequence as set forth in SEQ ID NO: 62; and a HCDR3 sequence comprising an amino acid sequence as set forth in SEQ ID NO: 63; or
      ii. a HCDR1 sequence comprising an amino acid sequence as set forth in SEQ ID NO: 70; a HCDR2 sequence comprising an amino acid sequence as set forth in SEQ ID NO: 71; and a HCDR3 sequence comprising an amino acid sequence as set forth in SEQ ID NO: 72; and
   b) the VL domain comprises an amino acid sequence that includes complementarity determining regions (CDRs):
      i. a LCDR1 sequence comprising an amino acid sequence as set forth in SEQ ID NO: 53; a LCDR2 sequence comprising an amino acid sequence as set forth in SEQ ID NO: 54; and a LCDR3 sequence comprising an amino acid sequence as set forth in SEQ ID NO: 55, or
      ii. a LCDR1 sequence comprising an amino acid sequence as set forth in SEQ ID NO: 79; a LCDR2 sequence comprising an amino acid sequence as set forth in SEQ ID NO: 80; and a LCDR3 sequence comprising an amino acid sequence as set forth in SEQ ID NO: 81.
39. The polypeptide according to any one of the preceding items, wherein the at least one anti-citrullinated protein antibody or fragment thereof comprises:
   a) a variable heavy chain (VH) domain comprising or consisting of an amino acid sequence selected from SEQ ID NO: 69 or SEQ ID NO: 78; and
   b) a variable light chain (VL) domain comprising or consisting of an amino acid sequence selected from SEQ ID NO: 60 or SEQ ID NO: 87.
40. The polypeptide according to any one of the preceding items, wherein the at least one anti-citrullinated protein antibody or fragment thereof is present in the sample of an individual with an autoimmune disease.
41. The polypeptide according to item 40, wherein the autoimmune disease is rheumatoid arthritis.
42. The polypeptide according to any one of items 40, wherein the sample is selected from the group comprising serum, plasma, saliva and synovial fluid.

### Modifications

43. The polypeptide according to any one of the preceding items, wherein the polypeptide further comprises a modification.
44. The polypeptide according to item 43, wherein the modification comprises incorporation of non-natural amino acids or D-amino acids, acetylation, phosphorylation, cyclization, stapling, conjugation to a biomolecule, chelation, lipidation, N-terminal acetylation, C-terminal amidation, PEGylation and/or glycosylation.
45. The polypeptide according to any one of items 43 to 44, wherein the modification comprises acetylation, phosphorylation, cyclization, stapling, conjugation to a biomolecule, chelation, lipidation, N-terminal.
46. The polypeptide according to any one of the preceding items, wherein the polypeptide is cyclic.
47. The polypeptide according to any of items 13-46, wherein the polypeptide is cyclised by a disulphide bridge between two cysteine residues.
48. The polypeptide according to item 47, wherein the disulfide bridge is formed between the cysteine residue nearest the N-terminus and the cysteine residue nearest the C-terminus of the polypeptide.
49. The polypeptide according to any one of the preceding items, wherein the modification comprises conjugation to a moiety selected from the group comprising: biotin or derivative thereof, an amino acid, a polypeptide, a protein, a carbohydrate, a lipid, a fatty acid, a triglyceride, a steroid, polyethylene glycol (PEG), or a fluorophore.
50. The polypeptide according to item 49, wherein the amino acid is an unnatural amino acid.
51. The polypeptide according to item 49, wherein the carbohydrate is selected from the group comprising a monosaccharide, disaccharide, or polysaccharide.
52. The polypeptide according to item 49, wherein the fatty acid is selected from the group comprising of saturated fatty acids and unsaturated fatty acids.
53. The polypeptide according to any one of the preceding items, wherein the modification comprises conjugation to a chemical tag, a protein tag, a fluorescent tag and/or an isotopic tag.
54. The polypeptide according to item 49, wherein the biotin moiety or derivative thereof is conjugated to the N-terminal end, the C-terminal end or an internal amino acid residue of the polypeptide.
55. The polypeptide according to any of items 49 and 54, wherein the biotin moiety or derivative thereof is conjugated to the N-terminal end of the polypeptide.
56. The polypeptide according to any of items 49, 54 and 55, wherein the biotin moiety or a derivative thereof is conjugated to the polypeptide via a linking moiety.
57. The polypeptide according to item 56, wherein the linking moiety is selected from the group comprising aminohexanoic acid (Ahx), one or more amino acids, one or more peptides, maleimide, polyethylene glycol (PEG) or oligoethylene glycol.
58. The polypeptide according to any of items 56-57, wherein the linking moiety is aminohexanoic acid (Ahx).
59. The polypeptide according to any one of the preceding items, wherein the polypeptide is selected from the group consisting of;
   SEQ ID NO: 130: Biotin-Ahx-CSKAKFAG-Cit-NFRNPLACA
   SEQ ID NO: 168: Biotin-Ahx-CVSPPVTV-Cit-GKEYPLGCA
   SEQ ID NO: 163: Biotin-Ahx-CVRVFQAT-Cit-GKLSSKCA
   SEQ ID NO: 179: Biotin-Ahx-CDFFTYHI-Cit-HGEVHCG, and
   SEQ ID NO: 120: Biotin-Ahx-CEKGVPLY-Cit-HIADLAGCA.
60. The polypeptide according to any one of the preceding items, wherein the polypeptide is SEQ ID NO: 130: Biotin-Ahx-CSKAKFAG-Cit-NFRNPLACA.
61. The polypeptide according to any one of the preceding items, wherein the polypeptide is SEQ ID NO: 168: Biotin-Ahx-CVSPPVTV-Cit-GKEYPLGCA.
62. The polypeptide according to any one of the preceding items, wherein the polypeptide is SEQ ID NO: 163: Biotin-Ahx-CVRVFQAT-Cit-GKLSSKCA.
63. The polypeptide according to any one of the preceding items, wherein the polypeptide is SEQ ID NO: 179: Biotin-Ahx-CDFFTYHI-Cit-HGEVHCG.
64. The polypeptide according to any one of the preceding items, wherein the polypeptide is SEQ ID NO: 120: Biotin-Ahx-CEKGVPLY-Cit-HIADLAGCA.

### Plurality of peptides

65. A plurality of polypeptides comprising at least two polypeptides as set forth in any of the preceding items.
66. A plurality of polypeptides comprising at least two polypeptides selected from the group comprising or consisting of SEQ ID NO: 1, 2, 3, 4, 5, 6, 7, 8, 9, 10, 11, 12, 13, 14, 15, 130, 168, 163, 179 and/or 120.
67. A plurality of polypeptides comprising at least two polypeptides selected from the group comprising or consisting of SEQ ID NO: 1, 2, 3, 4, 5, 6 and/or 7, such as SEQ ID NO: 3-7.
68. A plurality of polypeptides comprising at least two polypeptides selected from the group comprising or consisting of SEQ ID NO: 8, 9, 10, 11, 12, 13, 14 and/or 15, such as SEQ ID NO: 11, 12, 13, 14 and/or 15.
69. The plurality of polypeptides according to any one of the preceding items, wherein the plurality of polypeptides has a net positive charge or a net balanced charge.

### Composition

70. A composition comprising at least one polypeptide according to any one of the preceding items.

### Diagnosis/Prediction

71. A polypeptide for use in the diagnosis of autoimmune disease in a subject, wherein the polypeptide comprises or consists of a sequence selected from the group consisting of;
   SEQ ID NO: 3: SKAKFAG-Cit-NFRNPLA,
   SEQ ID NO: 4: VSPPVTV-Cit-GKEYPLG,
   SEQ ID NO: 5: VRVFQAT-Cit-GKLSSK,
   SEQ ID NO: 6: DFFTYHI-Cit-HGEVHCG, and
   SEQ ID NO: 7: EKGVPLY-Cit-HIADLAG.
72. A polypeptide for use in the prognosis of an autoimmune disease in an individual or identification of an individual at risk of developing an autoimmune disease, wherein the polypeptide comprises or consists of a sequence selected from the group consisting of;
   SEQ ID NO: 3: SKAKFAG-Cit-NFRNPLA,
   SEQ ID NO: 4: VSPPVTV-Cit-GKEYPLG,
   SEQ ID NO: 5: VRVFQAT-Cit-GKLSSK,
   SEQ ID NO: 6: DFFTYHI-Cit-HGEVHCG, and
   SEQ ID NO: 7: EKGVPLY-Cit-HIADLAG.
73. A polypeptide for use in the diagnosis of autoimmune disease in a subject, wherein the polypeptide is selected from the group consisting of;
   SEQ ID NO: 11: CSKAKFAG-Cit-NFRNPLACA,
   SEQ ID NO: 12: CVSPPVTV-Cit-GKEYPLGCA,
   SEQ ID NO: 13: CVRVFQAT-Cit-GKLSSKCA,
   SEQ ID NO: 14: CDFFTYHI-Cit-HGEVHCG, and
   SEQ ID NO: 15: CEKGVPLY-Cit-HIADLAGCA.
74. A polypeptide for use in the prognosis of an autoimmune disease in an individual or identification of an individual at risk of developing an autoimmune disease, wherein the polypeptide is selected from the group consisting of;
   SEQ ID NO: 11: CSKAKFAG-Cit-NFRNPLACA,
   SEQ ID NO: 12: CVSPPVTV-Cit-GKEYPLGCA,
   SEQ ID NO: 13: CVRVFQAT-Cit-GKLSSKCA,
   SEQ ID NO: 14: CDFFTYHI-Cit-HGEVHCG, and
   SEQ ID NO: 15: CEKGVPLY-Cit-HIADLAGCA.
75. A polypeptide for use in the diagnosis of autoimmune disease in a subject, wherein the polypeptide is selected from the group consisting of;
   SEQ ID NO: 130: Biotin-Ahx-CSKAKFAG-Cit-NFRNPLACA
   SEQ ID NO: 168: Biotin-Ahx-CVSPPVTV-Cit-GKEYPLGCA
   SEQ ID NO: 163: Biotin-Ahx-CVRVFQAT-Cit-GKLSSKCA
   SEQ ID NO: 179: Biotin-Ahx-CDFFTYHI-Cit-HGEVHCG, and
   SEQ ID NO: 120: Biotin-Ahx-CEKGVPLY-Cit-HIADLAGCA.
76. A polypeptide for use in the prognosis of an autoimmune disease in an individual or identification of an individual at risk of developing an autoimmune disease, wherein the polypeptide is selected from the group consisting of;
   SEQ ID NO: 130: Biotin-Ahx-CSKAKFAG-Cit-NFRNPLACA
   SEQ ID NO: 168: Biotin-Ahx-CVSPPVTV-Cit-GKEYPLGCA
   SEQ ID NO: 163: Biotin-Ahx-CVRVFQAT-Cit-GKLSSKCA
   SEQ ID NO: 179: Biotin-Ahx-CDFFTYHI-Cit-HGEVHCG, and
   SEQ ID NO: 120: Biotin-Ahx-CEKGVPLY-Cit-HIADLAGCA.
77. A polypeptide, plurality of polypeptides or composition according to any one of the preceding items for use in the diagnosis of an autoimmune disease in a subject.
78. A polypeptide, plurality of polypeptides or composition according to any one of the preceding items for use in the prognosis of an individual or identification of an individual at risk of developing an autoimmune disease.
79. A polypeptide, plurality of polypeptides or composition according to any one of the preceding items for use, wherein the autoimmune disease is rheumatoid arthritis.
80. The polypeptide, plurality of polypeptides or composition according to any one of the preceding items for use, wherein the polypeptide is used to detect the presence of at least one ACPA in a sample obtained from the individual, and wherein binding of the polypeptide or plurality of polypeptides to the at least one ACPAs is indicative of a reduced risk of disease severity, progression and/or activity.
81. The polypeptide, plurality of polypeptides or composition according to item 80, wherein the reduced risk of disease severity, progression and/or activity in the individual is quantified by improved clinical outcomes, such as a decreased tender joint count (TJC), decreased swollen joint count (SJC), decreased erythrocyte sedimentation rate (ESR), decreased C-reactive protein levels (CRP), and/or a reduced DAS28 score.
82. The polypeptide, plurality of polypeptides or composition according to any one of items 80-81, wherein the reduced risk of disease severity, progression and/or activity in the individual is measured by improved clinical outcomes, such as a decreased tender joint count (TJC), decreased swollen joint count (SJC), decreased erythrocyte sedimentation rate (ESR), decreased C-reactive protein levels (CRP), and/or a reduced DAS28 score, as compared to an individual wherein the ACPA present in the sample does not bind to the at least one polypeptide.
83. The polypeptide, plurality of polypeptides or composition according to any one of items 80 to 82 for use in prognosing the progression of an autoimmune disease in an individual, wherein the ACPA is as defined in any one of items 35-42.
84. The polypeptide, plurality of polypeptides or composition for use according to any one of items 77-83, wherein the diagnosis of a subject, prognosis of an individual or identification of an individual at risk of developing an autoimmune disease, such as rheumatoid arthritis, is determined before the onset of clinical symptoms, such as at the most 12 years before the onset of clinical symptoms, such as at the most 11 years before the onset of clinical symptoms, such as at the most 10 years before the onset of clinical symptoms, such as at the most 9 years before the onset of clinical symptoms, such as at the most 8 years before the onset of clinical symptoms, such as at the most 7 years before the onset of clinical symptoms, such as at the most 6 years before the onset of clinical symptoms, such as at the most 5 years before the onset of clinical symptoms, such as at the most 4 years before the onset of clinical symptoms, such as at the most 3 years before the onset of clinical symptoms, such as at the most 2 years before the onset of clinical symptoms, such as at the most 1 year before the onset of clinical symptoms, such as at the most 6 months before the onset of clinical symptoms; or at the onset of clinical symptoms; or after the onset of clinical symptoms, such as at the most 1 year after onset of clinical symptoms, such as at the least 1 year after onset of clinical symptoms.
85. The polypeptide, plurality of polypeptides or composition for use according to any one of items according to any of items 77-84, wherein the sample is selected from the group comprising serum, plasma, saliva and synovial fluid.

### Methods

86. A method of diagnosing rheumatoid arthritis in a subject, the method comprising exposing at least one polypeptide, plurality of polypeptides, or composition according to any one of items 1-58 to a sample obtained from said subject, and detecting whether at least one antibody present in the sample binds to the at least one polypeptide.
87. A method of diagnosing rheumatoid arthritis in a subject, the method comprising detecting whether at least one antibody present in a sample provided from the subject binds to at least one polypeptide according to any one of items 1-61.
88. A method of prognosing rheumatoid arthritis in an individual, or identifying an individual at risk of developing rheumatoid arthritis, the method comprising exposing at least one polypeptide, plurality of polypeptides, or composition according to any one of items 1-70 to a sample obtained from said individual, and detecting whether at least one antibody present in the sample binds to the at least one polypeptide.
89. A method of prognosing rheumatoid arthritis in an individual, or identifying an individual at risk of developing rheumatoid arthritis, the method comprising detecting whether at least one antibody present in a sample provided from the individual binds to at least one polypeptide, plurality of polypeptides, or composition according to any one of items 1-69.
90. A method of diagnosing rheumatoid arthritis in a subject, distinguishing between different types or stages of rheumatoid arthritis in a subject, prognosing rheumatoid arthritis in an individual or identifying an individual at risk of developing rheumatoid arthritis, the method comprising detecting whether at least one antibody present in a sample provided from the subject or individual, binds to to at least one polypeptide, plurality of polypeptides, or composition according to any one of items 1-69.
91. The method according to any one of items 86-90, the method comprising detecting whether at least one antibody present in a sample provided from the subject or individual, binds to at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120.
92. The method according to any one of items 86-91, the method comprising detecting whether at least one antibody present in a sample provided from the subject or individual, binds to at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the at least one polypeptide is immobilized on a surface.
93. A method of diagnosing rheumatoid arthritis in a subject, the method comprising the steps of:
   a) providing a sample obtained from the subject or individual,
   b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the at least one polypeptide is immobilized on a surface, and
   c) detecting whether at least one antibody present in the sample binds to the at least one polypeptide.
94. A method of distinguishing between different types or stages of rheumatoid arthritis (RA) in a subject, prognosing RA in an individual or identifying an individual at risk of developing RA, the method comprising the steps of:
   a) providing a sample obtained from the subject or individual,
   b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the at least one polypeptide is immobilized on a surface, and
   c) detecting whether at least one antibody present in the sample binds to the at least one polypeptide.
95. The method according to any one of items 93 to 94, the method comprising the steps of:
   a) providing a sample obtained from the subject or individual;
   b) contacting the sample with at least one polypeptide comprising or consisting of a sequence selected from the group consisting of SEQ ID NO: 130, 168, 163, 179 and 120, wherein the at least one polypeptide is immobilized on a surface, and
   c) detecting whether at least one antibody present in the sample binds to the at least one polypeptide.
96. A method for identifying an individual at risk of developing rheumatoid arthritis, the method comprising the steps of:
   a) providing a sample obtained from the individual;
   b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120wherein the at least one polypeptide is immobilized on a surface, and
   c) detecting whether at least one antibody present in the sample binds to the at least one polypeptide.
97. The method according to item 96, the method comprising the steps of:
   a) providing a sample obtained from the individual;
   b) contacting the sample with at least one polypeptide comprising or consisting of a sequence selected from the group consisting of SEQ ID NO: 130, 168, 163, 179 and 120wherein the at least one polypeptide is immobilized on a surface, and
   c) detecting whether at least one antibody present in the sample binds to the at least one polypeptide.
98. A method for the prognosis of rheumatoid arthritis in an individual, the method comprising the steps of:
   a) providing a sample obtained from the individual;
   b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the at least one polypeptide is immobilized on a surface, and
   c) detecting whether at least one antibody present in the sample binds to the at least one polypeptide, and
   d) prognosing a reduced severity, progression and/or activity of disease in the individual when the at least one antibody present in the sample is detected to bind to the at least one polypeptide.
99. The method according to any one of items 86-98, wherein the at least one antibody is as defined in any one of items 35-40.
100. The method according to any one of items 85-99, wherein the subject or individual is rheumatoid factor positive (seropositive).
101. The method according to any one of items 85-98, wherein the subject or individual is rheumatoid factor negative (seronegative).
102. The method according to any one of items 98-101, wherein the reduced severity, progression and/or activity of disease in the individual is quantified by improved clinical outcomes, such as a decreased tender joint count (TJC), decreased swollen joint count (SJC), decreased erythrocyte sedimentation rate (ESR), decreased C-reactive protein levels (CRP), and/or a reduced DAS28 score.
103. The method according to item 102, wherein the reduced severity, progression and/or activity of disease in the individual is measured by improved clinical outcomes, such as a decreased tender joint count (TJC), decreased swollen joint count (SJC), decreased erythrocyte sedimentation rate (ESR), decreased C-reactive protein levels (CRP), and/or a reduced DAS28 score, as compared to an individual wherein the antibody present in the sample does not bind to the at least one polypeptide.
104. The method according to any one of the preceding items, wherein the at least one polypeptide is immobilized on a surface, wherein the surface is solid.
105. The method according to any one of the preceding items, wherein at least one polypeptide is immobilized on a surface, wherein the surface comprises polypropylene, polystyrene, graphite, silica, glass, silicon, plastic, gold and/or microspheres.
106. The method according to any one of the preceding items, wherein the surface comprises microspheres, such as magnetic, polystyrene, glass, silica, polyacrylamide and/or latex beads.
107. The method according to any one of the preceding items, wherein the surface comprises avidin- and/or neutravidin-coated microspheres.
108. The method according to any one of the preceding items, wherein the at least one polypeptide is biotinylated and immobilized on a surface via a neutravidin-biotin interaction.

### Detection systems

109. The method according to any one of the preceding items, wherein detecting whether at least one antibody present in the sample binds to the at least one polypeptide comprises using a detection system selected from the group comprising a multiplex bead-based immunoassay system, flow cytometry, fluorescence-based detection, turbidimetry, radioisotope-based detection, and chemiluminescence-based detection.
110. The method according to item 109, wherein the detection system is selected from the group comprising an enzyme-linked immunosorbent assay (ELISA), chemiluminescence immunoassay (CLIA), lateral flow immunoassay (LFIA), immunoturbidimetric assay, radioimmunoassay (RIA), electrochemiluminescence (ECL) and electrochemiluminescence immunoassay (ECLIA).
111. The method according to any one of the preceding items, wherein the detecting whether the at least one antibody present in the sample binds to said at least one polypeptide comprises use of a detection reagent selected from the group consisting of enzyme-conjugated antibodies, fluorescence-conjugated antibodies, gold nanoparticles or luminescent probes.
112. The method according to any one of the preceding items, wherein the detection reagent is selected from the group comprising horseradish peroxidase (HRP), alkaline phosphatase (AP), R-phycoerythrin (R-PE), or chemiluminescent substrates.
113. The method according to any one of the preceding items, wherein detecting whether the at least one antibody present in the sample binds to said at least one polypeptide is quantified by measuring the signal intensity of the detection reagent.
114. The method according to any one of the preceding items, wherein detecting whether the at least one antibody present in the sample binds to said at least one polypeptide is quantified by measuring the signal intensity of the detection reagent, wherein the detection reagent is selected from the group comprising horseradish peroxidase (HRP), alkaline phosphatase (AP), R-phycoerythrin (R-PE), or chemiluminescent substrates.
115. The method according to any one of the preceding items, wherein;
   c. the at least one polypeptide is immobilized on a surface comprising a microplate coated with neutravidin, and
   d. detecting whether the at least one antibody present in the sample binds to said at least one polypeptide comprises using a detection system, wherein the detection system is an enzyme-linked immunosorbent assay (ELISA).
116. The method according to any one of the preceding items, wherein the detection system allows for simultaneous detection of the at least one antibody binding to a panel comprising at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120.
117. The method according to any one of the preceding items, wherein the detection system allows for simultaneous detection of the at least one antibody binding to a panel comprising at least one polypeptide comprising or consisting of a sequence selected from the group consisting of SEQ ID NO: 130, 168, 163, 179 and 120.
118. The method according any one of the preceding items, wherein the subject and/or individual is pre-symptomatic or symptomatic.
119. The method according to any one of the preceding items, wherein the subject and/or individual is identified before the onset of clinical symptoms, such as at the most 12 years before the onset of clinical symptoms, such as at the most 11 years before the onset of clinical symptoms, such as at the most 10 years before the onset of clinical symptoms, such as at the most 9 years before the onset of clinical symptoms, such as at the most 8 years before the onset of clinical symptoms, such as at the most 7 years before the onset of clinical symptoms, such as at the most 6 years before the onset of clinical symptoms, such as at the most 5 years before the onset of clinical symptoms, such as at the most 4 years before the onset of clinical symptoms, such as at the most 3 years before the onset of clinical symptoms, such as at the most 2 years before the onset of clinical symptoms, such as at the most 1 year before the onset of clinical symptoms, such as at the most 6 months before the onset of clinical symptoms; or at the onset of clinical symptoms; or after the onset of clinical symptoms, such as at the most 1 year after onset of clinical symptoms, such as at the least 1 year after onset of clinical symptoms.
120. The method according to any one of the preceding items, wherein the diagnosis in a subject, distinguishing between different types or stages of rheumatoid arthritis in a subject, or prognosis of rheumatoid arthritis in an individual is determined before the onset of clinical symptoms, such as at the most 12 years before the onset of clinical symptoms, such as at the most 11 years before the onset of clinical symptoms, such as at the most 10 years before the onset of clinical symptoms, such as at the most 9 years before the onset of clinical symptoms, such as at the most 8 years before the onset of clinical symptoms, such as at the most 7 years before the onset of clinical symptoms, such as at the most 6 years before the onset of clinical symptoms, such as at the most 5 years before the onset of clinical symptoms, such as at the most 4 years before the onset of clinical symptoms, such as at the most 3 years before the onset of clinical symptoms, such as at the most 2 years before the onset of clinical symptoms, such as at the most 1 year before the onset of clinical symptoms, such as at the most 6 months before the onset of clinical symptoms; or at the onset of clinical symptoms; or after the onset of clinical symptoms, such as at the most 1 year after onset of clinical symptoms, such as at the least 1 year after onset of clinical symptoms.
121. The method according to any one of the preceding items, wherein the method further comprises obtaining one or more clinical parameter.
122. The method according to item 121, wherein the one or more clinical parameter is utilised in combination with detecting whether at least one antibody present in the sample binds to the at least one polypeptide.
123. The method according to any one of items 121 to 122, wherein the one or more clinical parameter comprises clinical symptoms of rheumatoid arthritis; serological markers; X-ray; ultrasound; Magnetic Resonance Imaging (MRI); age and gender; family medical history, including rheumatoid arthritis or other autoimmune conditions; personal medical history, such as autoimmunity or joint trauma; genetic factors, including major histocompatibility complex, class II, DR beta 1 (HLA-DRB1), protein tyrosine phosphatase non-receptor type 22 (PTPN22), and signal transducer and activator of transcription 4 (STAT4); environmental factors, including smoking habits, infections, and hormonal changes; and patient-reported outcomes, such as interviews or questionnaires addressing pain, function, and quality of life.

### Companion diagnostics

124. A method for selecting an individual having rheumatoid arthritis (RA), the method comprising:
   a) providing a sample obtained from an individual;
   b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the polypeptide is immobilized on a surface,
   c) detecting whether at least one antibody present in the sample binds to the at least one polypeptide, and
   d) selecting the individual having RA following detection of binding of the at least one antibody present in the sample to the at least one polypeptide.
125. A method for selecting an individual at risk of developing rheumatoid arthritis (RA), the method comprising:
   a) providing a sample obtained from an individual, wherein the individual does not exhibit clinical symptoms of RA,
   b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the polypeptide is immobilized on a surface,
   c) detecting whether at least one antibody present in the sample binds to the at least one polypeptide, and
   d) selecting the individual having RA following detection of binding of the at least one antibody present in the sample to the at least one polypeptide.
126. The method according to any one of items 124-125, wherein the method further comprises administration of at least one therapeutic agent.
127. The method according to any one of items 124-126, wherein the at least one polypeptide comprising or consisting of a sequence selected from the group consisting of SEQ ID NO: 130, 168, 163, 179 and 120.
128. The method according to item 126, wherein the at least one therapeutic agent selected from the group comprising anti-inflammatory and immunosuppressive therapeutics, conventional Disease-Modifying Anti-Rheumatic Drugs (cDMARDs), biologic DMARDs (bDMARDs), Targeted Synthetic DMARDs (tsDMARDs); agents which modulate of regulatory T cells (Tregs), regulatory B cells (Bregs), or autoreactive T and/or B cells; Stem Cell Therapy, analgesics, and/or Peptidyl arginine deiminase 4 (PAD4) inhibitors.
129. The method according to item 128, wherein the anti-inflammatory and immunosuppressive therapeutics comprise non-steroidal anti-inflammatory Agents (NSAIDs), such as naproxen, indomethacin and/or COX-2 inhibitors, and/or corticosteroids.
130. The method according to item 128, wherein the conventional Disease-Modifying Anti-Rheumatic Drugs (cDMARDs) comprise methotrexate, sulfasalazine, hydroxychloroquine, and/or leflunomide.
131. The method according to item 128, wherein the biologic DMARDs (bDMARDs), comprise TNF Inhibitors, such as adalimumab, etanercept, infliximab, certolizumab pegol, and golimumab; IL-6 Inhibitors, such as tocilizumab or sarilumab; B-cell depleting antibodies, such as rituximab; T-cell Co-stimulation Modulators, such as abatacept; Interleukin-1 receptor antagonists (IL-1Ra), such as anakinra; otilimab, bispecific or trispecific antibodies which modulate immune response and/or inflammatory processes, and/or novel monoclonal antibodies, such as anti-citrullinated protein antibodies (ACPAs), such as CIT-013.
132. The method according to item 128, wherein the targeted synthetic DMARDs (tsDMARDs) comprise Janus Kinase (JAK) inhibitors, such as tofacitinib, baricitinib, upadacitinib, filgotinib, and peficitinib; and/or Bruton's Tyrosine Kinase (BTK) inhibitors, such as fenebrutinib.
133. The method according to item 128, wherein the agents which modulate of regulatory T cells (Tregs), suppressor B cells (Bsups), regulatory B cells (Bregs), or autoreactive T and/or B cells comprise agents which activate regulatory T cells or B cells, tolerogenic vaccines, and/or cell therapies for the modulation or expansion of the individual's regulatory cells.
134. The method according to item 128, wherein stem cell therapy comprises use of the individual's own mesenchymal stem cells (MSCs) for synovial MSC therapy.
135. The method according to any one of items 124-127 wherein the selection of the individual is determined based on the binding and/or binding affinity of the at least one antibody to the at least one polypeptide, wherein the binding and/or binding affinity of said antibody informs the selection of combination therapies of two or more therapeutic agents as set forth in any one of items 128-134.
136. The method according to any one of items 124-135, wherein the sample is selected from the group comprising serum, plasma, saliva and synovial fluid.

### Kit

137. A kit of parts comprising:
   a) at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120as defined in any of items 1-64, wherein the at least one polypeptide is immobilized on a surface; and
   b) at least one detection reagent selected from the group comprising enzyme-conjugated antibodies, fluorescence-conjugated antibodies, or luminescent probes for detecting antibody binding to the at least one polypeptide.
138. The kit of parts according to item 137, wherein the kit of parts comprises at least one polypeptide comprising or consisting of a sequence selected from the group consisting of SEQ ID NO: 130, 168, 163, 179 and 120.
139. The kit of parts according to any one of items 137-138, wherein the detection reagent comprises horseradish peroxidase (HRP), alkaline phosphatase (AP), fluorescent labels such as R-phycoerythrin (R-PE), radioisotopes, electrochemical labels, gold nanoparticles, quantum dots, bioluminescent enzymes or chemiluminescent substrates.
140. The kit of parts according to any of items 137-139, further comprising a secondary reagent for enhancing detection of antibody binding, wherein the secondary reagent is selected from the group comprising tyramide signal amplification (TSA) reagents, biotinylated antibodies, or streptavidin-conjugated probes.
141. The kit of parts according to any of items 137-140, wherein the surface comprises a material selected from the group comprising polypropylene, polystyrene, graphite, silica, glass, silicon, plastic, gold, and/or microspheres.
142. The kit of parts according to any one of items 137-141, further comprising at least one antibody as defined in any one of items 35-42.

### Examples

### Example 1 - Synthesis of peptides

### Aim

To synthesise polypeptides comprising citrulline, to serve as the anticipated epitope recognised by immunoglobulins, such as ACPAs, for capturing citrulline-specific antibodies. Non-citrullinated control peptides, with citrulline substituted by arginine, were also synthesised to differentiate between citrulline-specific antibodies and those binding to non-citrullinated peptides in tested samples.

### Materials and Methods

Amino acid sequences of the synthesised polypeptides were derived from the amino acid sequences of the human alpha enolase (ENO1; SEQ ID NO: 96) and human peptidyl arginine deiminase 4 (PAD4; SEQ ID NO: 97) full-length proteins. All arginine residues, potential sites of citrullination, were identified from the full-length amino acid sequences of the full-length ENO1 and PAD4 proteins and replaced with citrulline in the synthesised peptides. In addition, the corresponding non-citrullinated polypeptide, *i.e.,* comprising arginine, non-modified, were synthesised for each citrullinated polypeptide, such that pairs of citrullinated and corresponding non-citrullinated polypeptide were provided. The synthesised linear polypeptides consisted of 14 to 15 amino acids derived from the full-length ENO1 and PAD4 proteins.

The linear polypeptides were cyclised via the incorporation of a cysteine residue at both the N-terminus and C-terminus of the polypeptide. Said cysteine residues were not incorporated into the termini of the polypeptide if the linear polypeptide sequence already comprised of such termini cysteines. Other cysteine residues which were not present at either termini of the linear peptides, were replaced with serine to prevent internal disulfide formation. An additional neutral amino acid (alanine or glycine) was added to the C-terminus after the cysteine to stabilize the cyclic structure. Cyclisation was achieved via a disulphide bond between the cysteine residues at the N-terminus and C-terminus of the polypeptide.

At the N-terminus of the polypeptide, biotin was conjugated to each synthesised polypeptide via the flexible linker aminohexanoic acid (Ahx) (Liang *et al.* Rheumatology 2019).

The peptides were synthesized using solid phase chemistry, with disulfide bond formed by oxidation of cysteine thiol groups (-SH) to disulfide-bonds (-S-S-) using oxidizing agents such as iodine, hydrogen peroxide, or controlled air oxidation.

The final cyclic polypeptide structures were characterised and confirmed using analytical methods, including mass spectrometry for molecular mass determination, and purity (>90%) was verified by high-performance liquid chromatography (HPLC).

### Results

A series of 17 and 27 pairs of citrullinated and corresponding non-citrullinated polypeptides derived from ENO1 and PAD4, respectively, were successfully synthesised. All synthesised peptides are presented in Table 1. The amino acid sequences of all linear and cyclic polypeptides were confirmed by analytical methods including mass spectrometry (MS) or tandem mass spectrometry (MS/MS), with the observed molecular mass matching the theoretical mass of the designed sequences listed in Table 1, and their purity was determined to be greater than 90% using analytical techniques including high performance liquid chromatography (HPLC).

### Conclusion

Linear and cyclic citrullinated peptides covering all the possible citrullination sites on ENO1 and PAD4, as well as their non-citrullinated counterparts, were synthesised.

**Table 1. Synthesised linear and cyclic polypeptides. Pairs of citrullinated and corresponding non-citrullinated polypeptides derived from ENO1 and PAD4. Conformation denotes whether the polypeptide was linear or cyclic . 'R' denotes an arginine amino acid residue. 'CIT' denotes a citrulline amino acid residue.**

| **ENO1-derived peptides** | | | | | |
|---|---|---|---|---|---|
| | **Non-citrullinated** | | **Citrullinated** | | |
| **SEQ ID NO** | **Conformati on** | **Sequence** | **SEQ ID NO** | **Conformation** | **Sequence** |
| 98 | cyclic | Bio-Ahx-CSILKIHAREIFD SRGCA | 115 | cyclic | Bio-Ahx-CSILKIHA-Cit-EIFDSRGCA |
| 99 | cyclic | Bio-Ahx-CAREIFDSRGNP TVEVCA | 116 | cyclic | Bio-Ahx-CAREIFDS-Cit-GNPTVEVCA |
| 100 | cyclic | Bio-Ahx-CFTSKGLFRAAV PSGACA | 117 | cyclic | Bio-Ahx-CFTSKGLF-Cit-AAVPSGACA |
| 101 | cyclic | Bio-Ahx-CIYEALELRDND KTRYCA | 118 | cyclic | Bio-Ahx-CIYEALEL-Cit-DNDKTRYCA |
| 102 | cyclic | Bio-Ahx-CLRDNDKTRYM GK GVSCA | 119 | cyclic | Bio-Ahx-CLRDNDKT-Cit-YMGK GVSCA |
| 103 | cyclic | Bio-Ahx-CEKGVPLYRHIA DLAGCA | 120 | cyclic | Bio-Ahx-CEKGVPLY-Cit-HIADLAGCA |
| 104 | cyclic | Bio-Ahx-CPVGAANFREA MRIGACA | 121 | cyclic | Bio-Ahx-CPVGAANF-Cit-EAMRIGACA |
| 105 | cyclic | Bio-Ahx-CANFREAMRIGA EVYHCA | 122 | cyclic | Bio-Ahx-CANFREAM-Cit-IGAEVYHCA |
| 106 | cyclic | Bio-Ahx-CVAASE FFRSGKYDLDCA | 123 | cyclic | Bio-Ahx-CVAASEFF--Cit- -SGKYDLDCA |
| 107 | cyclic | Bio-Ahx-CKSPDDPSRYIS PDQLCA | 124 | cyclic | Bio-Ahx-CKSPDDPS-Cit-YISPDQLCA |
| 108 | cyclic | Bio-Ahx-CLTVTNPKRIAK AVNECA | 125 | cyclic | Bio-Ahx-CLTVTNPK-Cit-IAKAVNECA |
| 109 | cyclic | Bio-Ahx-CWGVMVSHRS GETEDTCA | 126 | cyclic | Bio-Ahx-CWGVMVSH-Cit-SGETEDTCA |
| 110 | cyclic | Bio-Ahx-CIKTGAPSRSER LAKYCA | 127 | cyclic | Bio-Ahx-CIKTGAPS-Cit-SERLAKYCA |
| 111 | cyclic | Bio-Ahx-CGAPSRSERLA KYNQLCA | 128 | cyclic | Bio-Ahx-CGAPSRSE-Cit-LAKYNQLCA |
| 112 | cyclic | Bio-Ahx-CAKYNQLLRIEE ELGSCA | 129 | cyclic | Bio-Ahx-CAKYNQLL-Cit-IEEELGSCA |
| 113 | cyclic | Bio-Ahx-CSKAKFAGRNF RNPLACA | 130 | cyclic | Bio-Ahx-CSKAKFAG-Cit-NFRNPLACA |
| 114 | cyclic | Bio-Ahx-CKAKFAGRNFR NPLAKCA | 131 | cyclic | Bio-Ahx-CKAKFAGRNF-Cit-NPLAKCA |

| **PAD4-derived peptides** | | | | | |
|---|---|---|---|---|---|
| | **Non-citrullinated** | | **Citrullinated** | | |
| **SEQ ID NO** | **Conformati on** | **Sequence** | **SEQ ID NO** | **Conformation** | **Sequence** |
| 132 | cyclic | Biotin-Ahx-CMAQGTLI RVTP EQPTCA | 157 | linear | Biotin-Ahx-MAQGTLI-Cit-VTPEQPTA |
| 133 | cyclic | Biotin-Ahx-CSLSADITRTGK VKPTCA | 158 | cyclic | Biotin-Ahx-CSLSADIT-Cit-TGKVKPTCA |
| 134 | cyclic | Biotin-Ahx-CTGKVKPTRAVK DQRTCA | 159 | cyclic | Biotin-Ahx-CTGKVKPT-Cit-AVKDQRTCA |
| 135 | cyclic | Biotin-Ahx-CTRAVKDQRTW TWGPCA | 160 | cyclic | Biotin-Ahx-CTRAVKDQ-Cit-TWTWGPCA |
| 136 | cyclic | Biotin-Ahx-CTLVLHVARSEM DKVRCA | 161 | cyclic | Biotin-Ahx-CTLVLHVA-Cit-SEMDKVRCA |
| 137 | cyclic | Biotin-Ahx-CRSEMDKVRVF QATRGCA | 162 | linear | Biotin-Ahx-RSEMDKV-Cit-VFQATRGA |
| 138 | cyclic | Biotin-Ahx-CVRVFQATRGK LSSKCA | 163 | cyclic | Biotin-Ahx-CVRVFQAT-Cit-GKLSSKCA |
| 139 | cyclic | Biotin-Ahx-CPVVFDSPRNR GLKEFCA | 164 | cyclic | Biotin-Ahx-CPVVFDSP-Cit-NRGLKEFCA |
| 140 | cyclic | Biotin-Ahx-CVFDSPRNRGL KEFPICA | 165 | cyclic | Biotin-Ahx-CVFDSPRN-Cit-GLKEFPICA |
| 141 | cyclic | Biotin-Ahx-CLKEFPIKRVMG PDFGCA | 166 | cyclic | Biotin-Ahx-CLKEFPIK-Cit-VMGPDFGCA |
| 142 | cyclic | Biotin-Ahx-CPDFGYVTRGP QTGGICA | 167 | cyclic | Biotin-Ahx-CPDFGYVT-Cit-GPQTGGICA |
| 143 | cyclic | Biotin-Ahx-CVSPPVTVRGK EYPLGCA | 168 | cyclic | Biotin-Ahx-CVSPPVTV-Cit-GKEYPLGCA |
| 144 | cyclic | Biotin-Ahx-CGKEYPLGRILF GDSCA | 169 | cyclic | Biotin-Ahx-CGKEYPLG-Cit-ILFGDSCA |
| 145 | cyclic | Biotin-Ahx-CYPSNDSRQMH QALQCA | 170 | cyclic | Biotin-Ahx-CYPSNDS-Cit-QMHQALQCA |
| 146 | cyclic | Biotin-Ahx-CSFVPAPDRKG FRLLLCA | 171 | cyclic | Biotin-Ahx-CSFVPAPD-Cit-KGFRLLLCA |
| 147 | cyclic | Biotin-Ahx-CAPDRKGFRLLL ASPRCA | 172 | cyclic | Biotin-Ahx-CAPDRKGF-Cit-LLLASPRCA |
| 148 | cyclic | Biotin-Ahx-CRLLLASPRSSY KLFQCA | 173 | cyclic | Biotin-Ahx-CRLLLASP-Cit-SSYKLFQCA |
| 149 | cyclic | Biotin-Ahx-CILSNKTLREHN SFVECA | 174 | linear | Biotin-Ahx-ILSNKTL-Cit-EHNSFVEA |
| 150 | cyclic | Biotin-Ahx-CEHNSFVERSID WNRECA | 175 | cyclic | Biotin-Ahx-CEHNSFVE-Cit-SIDWNRECA |
| 151 | cyclic | Biotin-Ahx-CERSIDWNRELL KRELCA | 176 | cyclic | Biotin-Ahx-CERSIDWN-Cit-ELLKRELCA |
| 152 | cyclic | Biotin-Ahx-CWNRELLKREL GLAESCA | 177 | cyclic | Biotin-Ahx-CWNRELLK-Cit-ELGLAESCA |
| 153 | cyclic | Biotin-Ahx-FGPVINGRSSLE EKVA | 178 | linear | Biotin-Ahx-FGPVING-Cit-SSLEEKVA |
| 154 | cyclic | Biotin-Ahx-CDFFTYHIRHGE VHCG | 179 | cyclic | Biotin-Ahx-CDFFTYHI-Cit-HGEVHCG |
| 155 | cyclic | Biotin-Ahx-CVHSGTNVRRK PFSFKCA | 180 | cyclic | Biotin-Ahx-CVHSGTNV-Cit-RKPFSFKCA |
| 156 | cyclic | Biotin-Ahx-CHSGTNVR**R**KP FSFKWCA | 181 | cyclic | Biotin-Ahx-CHSGTNVR-**Cit-**KPFSFKWCA |

### Example 2 - Reactivity of ACPAs to the citrullinated epitopes of the synthesised

### peptides

### Aim

To identify the specificities of monoclonal anti-citrullinated protein antibodies (ACPAs) derived from RA patients to the citrullinated epitopes of the synthesised peptides.

### Materials and Methods

All monoclonal ACPA (L1, L2, L4, L6, L8, L9, L10-L12 and 3F3WT ('non-protective' ACPAs); and E4NG and E4WT 'protective' ACPAs) were derived from single B cell clones isolated from CCP2-positive RA patients in the Rheumatology Department of Leiden University Medical Center. The B-cell receptors (BCRs) were sequenced and constructed with identical human IgG1 or mouse IgG2b constant domains. Rene E. M. Toes and Hans Ulrich Scherer (LUMC, The Netherlands) provided the sequence of L4 antibody clone.

The E4m antibody is the mutant of E4 antibody (W48M & S51A in paratope), lacking the citrulline-specificity and therefore it was used as a negative control for E4 (He *et al*.,2023). The E4NG antibody is a variant of E4 wildtype (E4WT) with the Fabglycosylation sites mutated, but the citrulline-specificity is maintained. All antibodies were produced in Expi293F cells, purified by affinity chromatography, dialyzed and stored in PBS at -20°C.

Antibody responses, *i.e.,* binding of at least one antibody to at least one polypeptide, were detected and analysed via a multiplex bead-based flow immunoassay (as described in Lönnblom *et al.,* 2023). At first, neutravidin (Thermo Fisher Scientific) was coupled to magnetic beads (MagPlex Microspheres, Luminex Corporation that can be distinguished by their original dyes. Thereafter, the biotinylated, synthesised cyclic citrullinated polypeptides derived from ENO1 and PAD4 (Table 1) were immobilized to the beads through avidin-biotin interaction. Cyclic citrullinated peptide 4 (CCP4) (Kissel *et al.,* 2019) and citrullinated alpha-enolase peptide 1 (CEP1) (Kinloch *et al.,* 2008) were utilised as positive controls. The corresponding non-citrullinated polypeptides were utilised as negative controls. After incubating the ACPAs ("L" series, E4NG, E4WT and 3F3WT) with the polypeptide-coupled beads, the antibodies bound to each polypeptide were detected utilising phycoerythrin-conjugated anti-human IgG Fcγ (Jackson Immunodiagnostics). Median fluorescence intensity (MFI) values were analysed via a Bio-Plex 200 System (Bio-Rad). Data integrity and/or result comparability between the plates was ensured by assay calibrators and control samples.

### Results

Antibody binding responses are presented in Figure 1. In contrast to the L1, L2, L4, L6, L8, L9, L10-L12 and 3F3WT antibodies ('non-protective' ACPAs) that recognised a limited number of citrullinated epitopes of the synthesised polypeptides, E4 (a 'protective' ACPA) bound to many citrullinated epitopes, indicating that the E4 'protective' ACPA could have a distinct pattern for epitope-recognition as compared with other 'non-protective' ACPAs.

### Conclusion

'Protective' ACPAs, such as antibody clone E4, have distinct binding patterns to citrullinated polypeptides in comparison with the 'non-protective' ACPAs, such as antibody clone L2.

### Example 3 - ACPA responses to the citrullinated epitopes of the synthesised polypeptides are present before onset of clinical symptoms and increase towards onset of RA

### Aim

To determine plasma IgG ACPA responses to the synthesised citrullinated and non-citrullinated polypeptides in pre-symptomatic individuals (before onset of clinical RA symptoms) and early RA patients (within 0-1 years of onset of clinical symptoms).

### Materials and Methods

Plasma samples were obtained from pre-symptomatic individuals (n= 520), who had donated a sample within 0-18 years before the onset of clinical RA symptoms. Of these individuals, 244 had also provided blood samples at the time of being diagnosed with RA thus creating paired samples. Plasma samples were obtained from the early RA patients (n= 244) within 0-1 years from the onset of clinical symptoms. Plasma samples were obtained from population controls (n= 530;), matched for age and sex with the pre-symptomatic individuals (Figure 3).

Antibody responses, i.e., binding of at least one antibody, or ACPA, to the synthesised polypeptides (Table 1) were analysed using the same multiplex bead-based flow immunoassay as described in Example 2. For each tested polypeptide, a cut-off MFI value was established, defined as the MFI value at which 5% of the control population had higher values. This cut-off was used to classify samples from pre-symptomatic individuals and early RA patients as positive antibody responses (MFI value above the cut-off) or negative antibody responses (MFI value equal to or below the cut-off) for the ACPA antibody response to the polypeptide. The frequencies of positive antibody responses were calculated for pre-symptomatic individuals, stratified by the number of years before RA onset, as well as for early RA patients for each tested polypeptide. The cumulative frequency of positive ACPA antibody responses in pre-symptomatic individuals relative to RA onset was determined as the ratio of positive samples collected within a specific time interval (in years before RA onset) or earlier, to the total number of samples collected during that same period.

### Results

The frequency of positive plasma ACPA antibody responses to the tested polypeptides are presented in Figure 2. Cumulative frequencies of antibody responses to the synthesised ENO1 and PAD4-derived polypeptides in samples from the pre-symptomatic individuals at timepoints before the RA onset (from 12 years to less than 1 year before RA onset), and in early RA are presented in Figure 2A (*i.e.,* total frequency until the given timepoint, starting from 12 years before RA onset). Frequencies of positive antibody responses to the same polypeptides in samples collected from the pre-symptomatic individuals within the specified time interval (from 5 years to less than 1 year before RA onset) before RA onset are presented in Figure 2B. Antibody (ACPA) responses to the synthesised citrullinated polypeptides derived from ENO1 and PAD4 in pre-symptomatic individuals was more frequent than the antibody responses to the corresponding non-citrullinated polypeptides. The frequency of the ACPA responses in pre-symptomatic individuals increased during the 5 years' period before RA onset and in early RA.

### Conclusion

ACPA responses to the synthesised citrullinated polypeptides derived from ENO1 and PAD4 in pre-symptomatic individuals increase in a time-dependent manner towards the onset of RA.

### Example 4 - ACPA fine specificities to the citrullinated epitopes of the synthesised polypeptides are associated with lower swollen joint counts (SJC) in early RA.

### Aim

To investigate the association between ACPA responses to the synthesised polypeptides and clinical outcomes in early RA (0-1 years after onset of clinical symptoms).

### Materials and Methods

Plasma samples from 244 patients with early RA were collected at the time of being diagnosed with RA and within 0-1 years after the onset of clinical symptoms and from population controls (n= 530) (Rantapää-Dahlqvist *et al.,* 2003). Samples were analysed for ACPA responses to the citrullinated epitopes of the synthesised polypeptides.

ACPA responses in the samples were determined and sample positivity for ACPAs to each given polypeptide were determined as described in Example 3. For each of the tested citrullinated polypeptides, the patients were categorised into being positive or negative for the ACPA response against the given citrullinated polypeptide.

The early RA patients' samples had been tested for the presence of RF as well as for the ACPAs (the anti-CCP2 test) using standard clinical laboratory tests (Rantapää-Dahlqvist *et al.,* 2003). Each patient was determined to be anti-CCP2 or RF positive or negative by comparing with the cut-off at 25 AU/ml and 20 AU/ml, respectively.

Clinical information of the early RA patients included C-reactive protein (CRP) and the 28-joint disease activity score (DAS28) and its components tender joint count (TJC), swollen joint count (SJC) and erythrocyte sedimentation rate (ESR) (Rantapää-Dahlqvist *et al.,* 2003).

The relationships between ACPA responses and clinical presentation of RA were analyzed by first comparing early RA patients positive vs negative for the serological biomarkers (including IgG ACPA responses against tested citrullinated peptides (positivity defined as in Example 3) as well as the anti-CCP2 (CCP2) and RF , . Statistical differences in clinical variables, including DAS28, TJC, SJC, ESR, and CRP values, between serological biomarker positive and (negative groups were assessed using Student's t-test. To quantify the change between the groups, the mean value of each clinical variable in the antibody-negative group was subtracted from the mean value in the antibody-positive group. The relative change was then calculated by comparing this difference to the mean value of the clinical variable in the antibody-negative group, and the relative change was expressed as a percentage. A negative relative change indicated a lower value for the clinical variable (e.g. a lower disease activity score) in the antibody-positive group compared to the antibody-negative group, while a positive relative change indicates a higher value (e.g. a higher disease activity score). Data analyses were performed on patients stratified based on their RF serological status into RF-positive and RF-negative patient groups.

### Results

Relative change in mean clinical presentation (DAS28, TJC, SJC, ESR and CRP) of early RA patients positive for the ACPA responses to the synthesised polypeptides compared to those negative for ACPA responses to the synthesised cyclic polypeptides is presented in Figure 4.

ACPA responses to several synthesised cyclic polypeptides, such as SEQ ID NO 115, SEQ ID NO 125, SEQ ID NO 130 and CCP4, were found to associate with lower SJC or TJC. Whereas SEQ ID NO 124 was associated with a higher ESR.

### Stratification by RF status

Stratification of early RA patients based on RF status (Figure 4) revealed an association between lower swollen joint counts (SJC) and the presence of ACPA fine specificities to eight citrullinated polypeptides, excluding CCP2, in RF-negative patients.

The strongest associations with lower swollen joint counts (SJC) were observed for ACPA fine specificities detected with the polypeptides SEQ ID NO: 163, SEQ ID NO: 127, SEQ ID NO: 130, and SEQ ID NO: 167, showing decreases of 52%, 39%, 43%, and 45%, respectively, between ACPA-positive and ACPA-negative patients in RF-negative early RA patients (Figure 4). Additionally, the presence of ACPA fine specificities for three polypeptides (SEQ ID NO: 163, SEQ ID NO: 167, and SEQ ID NO: 168) was associated with lower DAS28 scores in RF-negative early RA patients, with DAS28 reductions of 19%, 15%, and 16%, respectively, compared to patients negative for these specific ACPAs. Notably, such associations were not observed in RF-positive early RA patients.

No significant differences were observed in tender joint count (TJC), erythrocyte sedimentation rate (ESR), or C-reactive protein (CRP) levels between RF-negative patients and those with the measured ACPA fine specificities. However, there was a general trend indicating a "protective" association, as evidenced by a higher number of negative relative changes in the means of DAS28, TJC, SJC, ESR, and CRP in antibody-positive patients (106 out of 125 changes investigated) compared to positive relative changes (19 out of 125 changes) in RF-negative early RA patients (Figure 4).

In contrast, in the RF-positive group, ACPA responses to SEQ ID NO 121 and SEQ ID NO 124 were associated with higher DAS28 and ESR, respectively (Figure 4).

### Conclusion

In early RA, particularly in cases that are negative for RF, certain ACPA responses, are associated with lower SJC and/or disease activity (DAS28 score) especially for ACPA responses detected with synthesised polypeptides; SEQ ID NO 163, SEQ ID NO 167 and SEQ ID NO 168.

### Example 5 - ACPA fine specificities to the citrullinated epitopes of the synthesised polypeptides in pre-symptomatic individuals predict future RA disease activity at the onset of clinical symptoms

### Aim

To investigate whether ACPA fine specificities to the citrullinated epitopes of the synthesised polypeptides in pre-symptomatic individuals, can predict future RA disease activity at the onset of clinical symptoms.

### Materials and Methods

Samples were obtained from pre-symptomatic individuals (within 5 years before the onset of clinical symptoms, N=1 17 for DAS28 and ESR analysis; N=116 for SJC and TJC analysis; and N=82 for CRP analysis), whom were later diagnosed having RA. Control samples were obtained from matched population controls (n= 530) (Figure 3).

Samples were analysed for ACPA fine specificities as in Examples 3 and 4 with the panel of synthesised polypeptides in Figure 2A, and antibody positivity and antibody negativity was determined as described in Example 4. As the pre-symptomatic individuals were later diagnosed with RA, their clinical outcomes (SJC, TJC, DAS28, ESR and/or CRP) after disease onset were known. Thus, the ACPA responses to the citrullinated epitopes of the synthesised polypeptides (antibody positive or antibody negative), were determined at the pre-symptomatic phase and then subsequently their clinical presentation upon RA diagnosis and/or within 0-1 years of the onset of clinical symptoms, were analysed (Figure 5).

### Results

In samples from individuals obtained before the onset of RA clinical symptoms (within 5 years before the onset of clinical symptoms), ACPA responses to many of the synthesised citrullinated polypeptides were associated with improved clinical parameters (e.g., lower SJC, TJC, DAS28, ESR or CRP) upon RA diagnosis and/or within 0-1 years of the onset of clinical symptoms (Figure 5), including SEQ ID NO: 115, SEQ ID NO: 116, SEQ ID NO: 118, SEQ ID NO: 120, SEQ ID NO: 126, SEQ ID NO: 130, SEQ ID NO: 163, SEQ ID NO: 168, SEQ ID NO: 179 and CCP4. Patients positive for ACPA responses to most citrullinated polypeptides within the panel in their pre-symptomatic phase showed overall tendencies of having lower disease severity or RA upon RA diagnosis and/or within 0-1 years of the onset of clinical symptoms.

Several ACPA responses were notably associated with multiple clinical outcomes, particularly the polypeptides SEQ ID NO: 115, SEQ ID NO: 116, SEQ ID NO: 118, SEQ ID NO: 120, SEQ ID NO: 130, CCP4, SEQ ID NO: 168, and SEQ ID NO: 179. In particular, ACPA responses to SEQ ID NO: 130, SEQ ID NO: 163, and SEQ ID NO: 168 were linked to lower swollen joint counts (SJC) in RF-negative early RA patients (Figure 4). No significant associations were observed between the anti-CCP2 response and changes in clinical parameters, including DAS28, TJC, SJC, ESR, or CRP (Figure 5).

### Conclusion

ACPA responses to the polypeptides of the invention in pre-symptomatic individuals predict future RA prognosis at the onset of clinical symptoms.

### Example 6 - New ACPA test predicts disease activity at RA onset

### Aim

To develop a new, superior ACPA test to detect specific ACPA responses in samples from pre-symptomatic individuals and to evaluate whether the new test could improve the diagnostic and/or prognostic and/or predictive value of the clinical test for ACPA, the anti-CCP2 test.

### Materials and Methods

Plasma samples collected from early RA patients (n=244) and pre-symptomatic individuals (n=287) were obtained. Samples from population controls (n=530) matched for age and sex with the pre-symptomatic individuals (Figure 3) were also obtained. All samples were evaluated for ACPA responses, *i.e.,* binding of plasma IgGs to each of the polypeptides in Figure 2A and detecting and analysing via a multiplex bead-based flow immunoassay as median fluorescence intensity (MFI) as in Example 3.

For the new ACPA test, five candidate polypeptides (SEQ ID NO: 120, SEQ ID NO: 130, SEQ ID NO: 163, SEQ ID NO: 168 and SEQ ID NO: 179) were selected based on their ACPA responses being associated with a less severe RA prognosis, as defined as the decrease of the mean clinical presentation of DAS28, TJC, SJC, ESR and/or CRP in ACPA response positive cases vs ACPA response negative cases, as evaluated in samples collected at pre-symptomatic stage and/or early RA. As demonstrated in the examples herein, the selected five polypeptides displayed both diagnostic value in early RA cases and predictive value in pre-symptomatic individuals, and predictive value in predicting different clinical presentation of DAS28, TJC, SJC, ESR and/or CRP.

For each tested polypeptide, the cut-off MFI value was defined as the MFI value above which 5% of the control population samples fell. This cut-off was used to classify each sample as either positive (MFI value above the cut-off) or negative (MFI value equal to or below the cut-off) for the IgG antibody response to the respective polypeptide.

The frequencies of positive ACPA responses to each of the selected polypeptides were calculated as the percentage of individuals with positive responses, separately for each group: early RA patients, pre-symptomatic individuals, and population controls. For each group, the number of positive ACPA responses to the five selected polypeptides was determined for each individual. The frequency of individuals showing positivity to 0, 1 or more (≥1), 2 or more (≥2), 3 or more (≥3), 4 or more (≥4), and all 5 polypeptides (=5) was then calculated separately for each group. The frequencies of positive ACPA responses are presented in Figure 6A and Table 2.

Pre-symptomatic individuals sampled 0 to 5 years before RA onset were categorised based on the number of positive ACPA responses to the five selected polypeptides into groups with ≥1, ≥2, ≥3, ≥4, or all 5 positive responses, as well as those with no response (=0). The clinical parameters at their future RA diagnosis during the early RA stage, including DAS28, ESR, TJC, SJC, and CRP, were then compared across the groups with varying numbers of positive ACPA responses (Figure 6B-F).

Anti-CCP2 (CCP2) levels were measured using enzyme-linked immunoassays (ELISA) in accordance with the manufacturer's instructions (Euro-Diagnostica AB, Sweden) (Rantapää-Dahlqvist *et al.,* 2003), with a cut-off value of 25 AU/ml used to define a positive result.

To compare the diagnostic, prognostic, and/or predictive value of the clinical anti-CCP2 test with the new ACPA test, an analysis was conducted to determine whether the frequency of positive results for the new ACPA test in early RA patients and/or pre-symptomatic individuals was significantly higher than in population controls, specifically among those who were negative for the anti-CCP2 test. If the new ACPA test showed a higher frequency of positivity in these groups compared to controls, it was considered to have superior diagnostic value over the anti-CCP2 test.

Additionally, to compare the prognostic and/or predictive value of the two tests, the differences in clinical presentation (DAS28, ESR, TJC, SJC, and CRP) between groups that were positive or negative for the anti-CCP2 test, and between groups categorized by the number of positive ACPA responses (≥1, ≥2, ≥3, ≥4, or =5 vs. =0), were calculated. The statistical significance of these differences was assessed with Mann-Whitney U test. If the new ACPA test demonstrated statistically significant differences in clinical outcomes, whereas the anti-CCP2 test did not, the new ACPA test was deemed superior in terms of prognostic or predictive value.

### Results

Compared to the anti-CCP2 test, which showed a sensitivity of 77.6% in early RA and 41% in pre-symptomatic individuals at a specificity of 98%, the panel of five polypeptides demonstrated higher sensitivities. Specifically, the polypeptides exhibited a sensitivity of 77.0% in early RA and 42.9% in pre-symptomatic individuals, with a specificity of 94.7% when 2 or more (≥2) ACPA responses were detected (Table 2). Furthermore, sensitivity was increased to 88.3% in RF-positive early RA patients, 39.3% in RF-negative early RA patients, and 53.7% in pre-symptomatic individuals within 0 to 5 years before the onset of clinical RA symptoms (Figure 6A).

Among anti-CCP2-negative individuals, who had tested negative with the currently clinically used ACPA test, 31.5% of early RA patients and 29.1% (95% Cl: 24% - 34%) of pre-symptomatic individuals were identified as positive for at least one of the five ACPA responses (≥1) (Table 3). The new ACPA test more accurately diagnosed RA in early RA patients compared to population controls when individuals tested positive for one or more (≥1) of the five ACPA responses. Additionally, the new ACPA test more frequently predicted future RA onset in pre-symptomatic individuals than in controls, when individuals were positive for one or more (≥1), or two or more (≥2) of the five ACPA responses, as evidenced by the non-overlapping confidence intervals (Table 3). The new ACPA test was found to be superior to the currently clinically used anti-CCP2 test, as the anti-CCP2 test failed to identify any of the early RA patients, population controls, or pre-symptomatic individuals at risk of future RA onset in this population.

For samples positive for 2 to 5 (≥2) ACPA responses, 11.1% early RA patients and 10.1% of pre-symptomatic individuals were detected with a specificity of 96% (Table 3). Significantly more pre-symptomatic individuals vs controls tested positive with the new ACPA test (Table 3).

Furthermore, individuals who exhibited positive ACPA responses to ≥1, ≥2, ≥3, ≥4, or all 5 of the polypeptides in the new ACPA test demonstrated significantly lower disease activity (as measured by DAS28, ESR, TJC, SJC, and CRP) compared to those who had no ACPA response (0) to any of the five selected polypeptides. A clear decreasing trend in disease activity was observed as the number of positive ACPA responses increased (Figure 6B-F). Notably, the anti-CCP2 test did not provide predictive value for any of these outcomes (Figure 5).

**Table 2. Comparison of the CCP2 test and the new ACPA test. Positivity for the at least 2 of the five polypeptides is considered a positive test result. Sensitivity and specificity are shown with 95% confidence intervals.**

| | **Early RA (N=244)** | | **Presymptomatic (N=520)** | | **Controls (N=530)** | |
|---|---|---|---|---|---|---|
| **Test** | **Test positive n (%)** | **Test negative n (%)** | **Test positive n (%)** | **Test negative n (%)** | **Test positive n (%)** | **Test negative n (%)** |
| RF | 188/244 (77; 72, 82) | 56/244 (23) | 189/513 (37; 33, 41) | 324/513 (63) | 33/251 (13) | 218/251 (87; 83, 91) |
| CCP2 | 187/241 (78; 72, 83) | 54/241 (22) | 213/519 (41; 37, 45) | 306/519 (59) | 16/530 (3) | 514/530 (97; 96, 98) |
| RF+ and/or CCP2+ | 210/244 (86; 82, 90) | 34/244 (14;10, 18) | 256/519 (49; 45, 54) | 263/519 (51;46, 55) | 47/525 (9; 7,11) | 478/525 (91;89, 93) |
| ≥2 Ab⁺ * | 188/244 (77; 72, 82) | 56/244 (23) | 223/520 (43; 39, 47) | 297/520 (57) | 28/530 (5) | 502/530 (95; 93, 97) |
| ≥3 Ab⁺ * | 171/244 (70; 64, 76) | 73/244 (30) | 187/520 (36; 32, 40) | 333/520 (64) | 19/530 (4) | 511/530 (96; 95, 98) |

| | | | | | | |
|---|---|---|---|---|---|---|
| *Ab⁺, positive ACPA response to the selected polypeptides, SEQ ID NO: 120, SEQ ID NO: 130, SEQ ID NO: 163, SEQ ID NO: 168 and SEQ ID NO: 179. | | | | | | |

**Table 3. Performance of the new ACPA test in anti-CCP2-negative individuals. Positivity for at least 1 or 2 (≥1 or ≥2) of the five polypeptides was considered a positive test result for the new ACPA test. Sensitivity and specificity are shown with 95% confidence intervals.**

| | **Early RA (N=54)** | | **Presymptomatic (N=306)** | | **Controls (N=514)** | |
|---|---|---|---|---|---|---|
| **Test** | **Test positive n (%)** | **Test negative n (%)** | **Test positive n (%)** | **Test negative n (%)** | **Test positive n (%)** | **Test negative n (%)** |
| RF | 20/54 (37; 24, 50) | 34/54 (63; 50, 76)) | 43/303 (14; 10, 18) | 260/303 (86; 82, 90) | 31/243 (13;9, 17) | 212/243 (87; 83, 91) |
| CCP2 | 0/54 (0; 0, 0) | 54/54 (100) | 0/306 (0; 0, 0) | 306/306 (100) | 0/514 (0) | 514/514 (100; 100, 100) |
| ≥1 Ab⁺ * | 17/54 (31; 19, 44) † | 37/54 (69) | 89/306 (29; 24, 34) † | 217/306 (71) | 67/514 (13) | 447/514 (87; 84, 89) |
| ≥2 Ab⁺ * | 6/54 (11; 3, 19) | 48/54 (89) | 31/306 (10; 7, 14) † | 275/306 (90) | 21/514 (4) | 493/514 (96; 94, 98) |

| | | | | | | |
|---|---|---|---|---|---|---|
| *Ab⁺, positive ACPA response to the selected polypeptides, SEQ ID NO: 120, SEQ ID NO: 130, SEQ ID NO: 163, SEQ ID NO: 168 and SEQ ID NO: 179. † Non-overlapping 95% confidence interval is considered statistically significant compared with population control. | | | | | | |

### Conclusion

The five selected polypeptides have an improved diagnostic and prognostic capacity compared with the anti-CCP2 test, as demonstrated by increased sensitivity and specificity. As such, they possess unique predictive capabilities in pre-symptomatic individuals for the disease activity at future onset. In individuals which were anti-CCP2-negative, the five selected polypeptides had the capacity to accurately diagnose RA in both early RA patients and pre-symptomatic individuals.

The selected five polypeptides exhibit both diagnostic value in early RA cases and predictive value in pre-symptomatic individuals, and predictive value in predicting different clinical presentation of DAS28, TJC, SJC, ESR and/or CRP. The peptides, both when utilised alone or synergistically, exhibit the capacity to accurately predict and/or prognose RA patients before the onset of clinical symptoms, and diagnose RA patients. Said improvements are further enhanced in RF-positive early RA patients, RF-negative early RA patients and in pre-symptomatic RA individuals.

### References

He Y, et al., Shift in perspective: autoimmunity protecting against rheumatoid arthritis. Annals of the Rheumatic Diseases 2024;83:550-555.
He, Y., et al., A subset of antibodies targeting citrullinated proteins confers protection from rheumatoid arthritis. Nat Commun 14, 691 (2023). https://doi-org.proxy.kib.ki.se/10.1038/s41467-023-36257-x
Lönnblom E, et al., Autoantibodies to Disease-Related Proteins in Joints as Novel Biomarkers for the Diagnosis of Rheumatoid Arthritis. Arthritis Rheumatol. 2023 Jul;75(7):1110-1119. doi: 10.1002/art.42463. Epub 2023 Apr 27. PMID: 36718635.
Rantapää-Dahlqvist S, et al., Antibodies against cyclic citrullinated peptide and IgA rheumatoid factor predict the development of rheumatoid arthritis. Arthritis Rheum. 2003 Oct;48(10):2741-9. doi: 10.1002/art.11223. PMID: 14558078.
Liang et al., The autoantibody response to cyclic citrullinated collagen type II peptides in rheumatoid arthritis, Rheumatology, Volume 58, Issue 9, September 2019, Pages 1623-1633, https://doi.org/10.1093/rheumatology/kez073
Kissel T, van Schie KA, Hafkenscheid L, Lundquist A, Kokkonen H, Wuhrer M, Huizinga TW, Scherer HU, Toes R, Rantapää-Dahlqvist S. On the presence of HLA-SE alleles and ACPA-IgG variable domain glycosylation in the phase preceding the development of rheumatoid arthritis. Ann Rheum Dis. 2019 Dec;78(12):1616-1620. doi: 10.1136/annrheumdis-2019-215698. Epub 2019 Aug 30. PMID: 31471298.
Kinloch A, Lundberg K, Wait R, Wegner N, Lim NH, Zendman AJ, Saxne T, Malmström V, Venables PJ. Synovial fluid is a site of citrullination of autoantigens in inflammatory arthritis. Arthritis Rheum. 2008 Aug;58(8):2287-95. doi: 10.1002/art.23618. PMID: 18668562.
Aletaha D, et al., 2010 Rheumatoid arthritis classification criteria: an American College of Rheumatology/European League Against Rheumatism collaborative initiative. Arthritis Rheum. 2010 Sep;62(9):2569-81. doi: 10.1002/art.27584. PMID: 20872595.
Smolen J et al., EULAR recommendations for the management of rheumatoid arthritis with synthetic and biological disease-modifying antirheumatic drugs: 2022 update. Ann Rheum Dis. 2023 Jan;82(1):3-18. doi: 10.1136/ard-2022-223356. Epub 2022 Nov 10. Erratum in: Ann Rheum Dis. 2023 Mar;82(3):e76. doi: 10.1136/ard-2022-223356corr1. PMID: 36357155.

## Claims

1. A polypeptide comprising or consisting of a sequence selected from the group consisting of:
SEQ ID NO: 3: SKAKFAG-Cit-NFRNPLA
SEQ ID NO: 4: VSPPVTV-Cit-GKEYPLG
SEQ ID NO: 5: VRVFQAT-Cit-GKLSSK, and
SEQ ID NO: 6: DFFTYHI-Cit-HGEVHCG

2. The polypeptide according to claim 1, wherein the polypeptide comprises of consists of a sequence selected from the group consisting of:
SEQ ID NO: 11: CSKAKFAG-Cit-NFRNPLACA
SEQ ID NO: 12: CVSPPVTV-Cit-GKEYPLGCA
SEQ ID NO: 13: CVRVFQAT-Cit-GKLSSKCA
SEQ ID NO: 14: CDFFTYHI-Cit-HGEVHCG, and
SEQ ID NO: 15: CEKGVPLY-Cit-HIADLAGCA

3. The polypeptide according to any one of the preceding claims, wherein the polypeptide consists of between 12 to 20 amino acid residues.

4. The polypeptide according to any one of the preceding claims, wherein the polypeptide is cyclic.

5. The polypeptide according to any one of the preceding claims, wherein the polypeptide comprises a biotin moiety.

6. The polypeptide according to claim 5, wherein the biotin moiety is conjugated to the polypeptide via a linking moiety selected from the group comprising aminohexanoic acid (Ahx), one or more amino acids, one or more peptides, maleimide, polyethylene glycol (PEG) or oligoethylene glycol.

7. A plurality of polypeptides comprising at least two polypeptides as set forth in any of the preceding claims.

8. The polypeptide or plurality of polypeptides according to any one of the preceding claims for use in the diagnosis of a subject, prognosis of an individual or identification of an individual at risk of developing an autoimmune disease.

9. The polypeptide or plurality of polypeptides for use according to claim 8, wherein the diagnosis of a subject, prognosis of an individual or identification of an individual at risk of developing an autoimmune disease comprises use of the polypeptide or plurality of polypeptides to detect the presence of at least one ACPA in a sample obtained from the subject or individual, and wherein binding of the polypeptide or plurality of polypeptides to the at least one ACPA indicates a reduced risk of disease severity, progression and/or activity.

10. The polypeptide or plurality of polypeptides for use according to claim 9, wherein the reduced risk of disease severity, progression and/or activity in the subject or individual is quantified by improved clinical outcomes, such as a decreased tender joint count (TJC), decreased swollen joint count (SJC), decreased erythrocyte sedimentation rate (ESR), decreased C-reactive protein levels (CRP), and/or a reduced DAS28 score, as compared to a subject or individual wherein the ACPA present in the sample does not bind to the at least one polypeptide.

11. A method of diagnosing rheumatoid arthritis in a subject, the method comprising detecting whether at least one ACPA present in a sample provided from the subject or individual, binds to at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120.

12. A method of prognosing rheumatoid arthritis in an individual or identifying an individual at risk of developing rheumatoid arthritis, the method comprising detecting whether at least one ACPA present in a sample provided from the subject or individual, binds to at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120.

13. The method according to any one of claims 11 and 12, wherein the method comprises the steps of:
a) providing a sample obtained from the subject or individual,
b) contacting the sample with at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the at least one polypeptide is immobilized on a surface, and
c) detecting whether at least one ACPA present in the sample binds to the at least one polypeptide.

14. The method according to any one of claims 11 to 13, wherein the subject or individual is rheumatoid factor positive (seropositive).

15. A kit of parts comprising:
a) at least one polypeptide comprising or consisting of a sequence selected from the group consisting of SEQ ID NO: 1, 2, 3, 4, 5, 6, 7, 8, 9, 10, 11, 12, 13, 14, 15, 130, 168, 163, 179 and 120, wherein the at least one polypeptide is immobilized on a surface; and,
b) at least one detection reagent selected from the group comprising enzyme-conjugated antibodies, fluorescence-conjugated antibodies, or luminescent probes for detecting antibody binding to the at least one polypeptide.
